# EUROPEAN PATENT SPECIFICATION

(11) **EP 2 946 323 B1**
(45) Date of publication and mention of the grant of the patent: **26.02.2020**
(21) Application number: 14740322.4
(22) Date of filing: 21.01.2014
(51) Int. Cl.: G16H 10/60, G06Q 50/22

(54) **SECURE REAL-TIME HEALTH RECORD EXCHANGE**
SICHERER ECHTZEITAUSTAUSCH VON GESUNDHEITSAKTEN
ÉCHANGE SÉCURISÉ DE DOSSIERS MÉDICAUX EN TEMPS RÉEL

(30) Priority: 21.01.2013 US 201361754916 P; 18.07.2013 US 201361847992 P
(43) Date of publication of application: 25.11.2015
(73) Proprietor: Humetrix.com, Inc., Del Mar, CA 92014 (US); Experton, Bettina, Del Mar, CA 92014 (US)
(72) Inventor: EXPERTON, Bettina, Del Mar, CA 92014 (US)
(74) Representative: HGF Limited
(86) International application number: PCT/US2014/012401
(87) International publication number: WO 2014/113817

(56) References cited:
- US-A1- 2010 094 658
- US-A1- 2010 250 271
- US-A1- 2011 282 688
- US-A1- 2012 232 929
- US-A1- 2012 232 929
- Lisa A. Eramo: "When Is It Time to Part Ways With Medical Records?", , 18 June 2012 (2012-06-18), XP002767304, Retrieved from the Internet: URL:http://www.fortherecordmag.com/archive s/061812p14.shtml [retrieved on 2017-02-15]

## Description

### BACKGROUND

### Field

The present invention relates generally to electronic healthcare records and more particularly to access and exchange of electronic healthcare records using mobile computing devices.

### Background

In today's healthcare environment individuals typically receive healthcare from multiple healthcare providers and often at multiple locations. Healthcare providers commonly lack accurate and up-to-date information regarding the care previously received by a patient from other providers. In order to deliver optimum, coordinated healthcare and most cost-effective healthcare to their patients, healthcare providers need to have ready access to an up to date medical history of their patients wherever they have received care, and the ability to exchange their most recent clinical findings and treatment plans to other healthcare providers who will be caring for their patients next.

To deliver such optimum care coordinated healthcare, new healthcare delivery and financing models have been defined, which emphasize coordination of care with the use of patient-centered medical homes (PCMHs) or accountable care organizations (ACOs). Implementation of such systems, however, can require significant changes in clinical practice and can result increased complexity in business, financing and contractual arrangements associated with the delivery and receipt of medical services. Healthcare information technology (HIT) systems are also now been developed and used to improve care coordination. HIT systems may include regional, federal and state health information exchanges (HIEs), provider-to-provider connectivity solutions using the nationwide health information network (NwHIN) and Direct protocol, or proprietary systems. However, such HIT solutions can be complex and costly to install and operate, and their use by providers (e.g. physicians) can be time-consuming and cumbersome, and often leave connectivity gaps between systems and providers.

US-2010/250271-A1 discloses a patient-initiated e-Visit to address a healthcare issue with an issue-focused adaptive interview, within the context of a digital healthcare platform. The results of this adaptive interview are forwarded to a skilled clinician for review, who then provides an assessment and a plan of action for the issue. The plan of action may include specific instructions, a prescription, or a referral to a third party medical provider for testing, consultation, or treatment. Another embodiment provides an identification "ticket" to the patient to coordinate care obtained at third parties. The ticket can be presented by the patient to a third party medical provider (such as with a barcode displayed on a mobile device) to identify the patient and enable the third party medical provider to access patient information from the digital healthcare platform.

### SUMMARY

The scope of the present invention is defined by the appended claims.

### BRIEF DESCRIPTION OF THE DRAWINGS

FIG. 1 is a diagram illustrating an example of a hardware implementation for an apparatus employing a processing system.
FIG. 2 is a block diagram illustrating an example of an electronic records delivery system according to certain aspects of the invention.
FIG. 3 is a conceptual diagram illustrating flow of electronic health records between a patient and physicians.
FIG. 4 illustrates a first example of proximity exchange between client and provider devices according to certain aspects of the invention.
FIG. 5 illustrates a second example of proximity exchange between client and provider devices according to certain aspects of the invention.
FIG. 6 illustrates a simplified example of the delivery of medical records to users of systems deployed according to certain aspects of the invention.
FIG. 7 includes flowcharts illustrating certain aspects of health record exchanges as described herein.
FIG. 8 is a diagram illustrating a first simplified example of a hardware implementation for an apparatus employing a processing system configured to perform certain functions according to certain aspects of the invention.
FIG. 9 is a diagram illustrating a second simplified example of a hardware implementation for an apparatus employing a processing system configured to perform certain functions according to certain aspects of the invention.

### DETAILED DESCRIPTION

The detailed description set forth below in connection with the appended drawings is intended as a description of various configurations and is not intended to represent the only configurations in which the concepts described herein may be practiced. The detailed description includes specific details for the purpose of providing a thorough understanding of various concepts. However, it will be apparent to those skilled in the art that these concepts may be practiced without these specific details. In some instances, well known structures and components are shown in block diagram form in order to avoid obscuring such concepts.

Several aspects of records management systems will now be presented with reference to various apparatus and methods. These apparatus and methods will be described in the following detailed description and illustrated in the accompanying drawing by various blocks, modules, components, circuits, steps, processes, algorithms, etc. (collectively referred to as "elements"). These elements may be implemented using electronic hardware, computer software, or any combination thereof. Whether such elements are implemented as hardware or software depends upon the particular application and design constraints imposed on the overall system.

By way of example, an element, or any portion of an element, or any combination of elements may be implemented with a "processing system" that includes one or more processors. Examples of processors include microprocessors, microcontrollers, digital signal processors (DSPs), field programmable gate arrays (FPGAs), programmable logic devices (PLDs), state machines, gated logic, discrete hardware circuits, and other suitable hardware configured to perform the various functionality described throughout this disclosure. One or more processors in the processing system may execute software. Software shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise. The software may reside on a computer-readable medium. A computer-readable medium may include, by way of example, a magnetic storage device (e.g., hard disk, floppy disk, magnetic strip), an optical disk (e.g., compact disk (CD), digital versatile disk (DVD)), a smart card, a flash memory device (e.g., card, stick, key drive), Near Field Communications (NFC) token, random access memory (RAM), read only memory (ROM), programmable ROM (PROM), erasable PROM (EPROM), electrically erasable PROM (EEPROM), a register, a removable disk, a carrier wave, a transmission line, and any other suitable medium for storing or transmitting software. The computer-readable medium may be resident in the processing system, external to the processing system, or distributed across multiple entities including the processing system. Computer-readable medium may be embodied in a computer-program product. By way of example, a computer-program product may include a computer-readable medium in packaging materials. Those skilled in the art will recognize how best to implement the described functionality presented throughout this disclosure depending on the particular application and the overall design constraints imposed on the overall system.

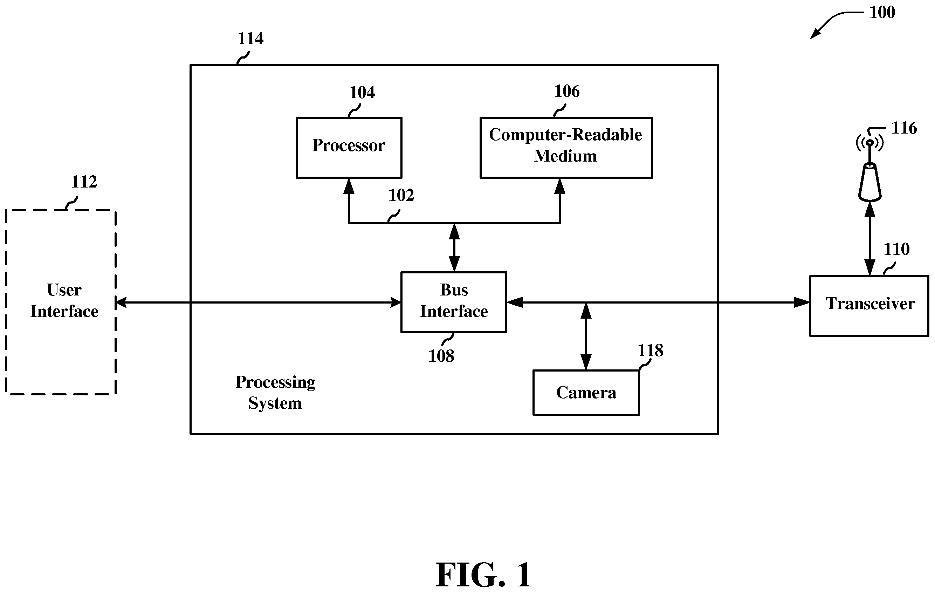

FIG. 1 is a conceptual diagram illustrating an example of a hardware implementation for an apparatus 100 employing a processing system 114. In this example, the processing system 114 may be implemented with a bus architecture, represented generally by the bus 102. The bus 102 may include any number of interconnecting buses and bridges depending on the specific application of the processing system 114 and the overall design constraints. The bus 102 links together various circuits including one or more processors, represented generally by the processor 104, and computer-readable media, represented generally by the computer-readable medium 106. The bus 102 may also link various other circuits such as timing sources, peripherals, voltage regulators, and power management circuits, which are well known in the art, and therefore, will not be described any further. A bus interface 108 may provide an interface between the bus 102 and certain peripherals, such as transceiver 110 and camera 118. In some embodiments, bus interface 108 may be an integral part of processor 104. In some embodiments, bus interface 108 may interface a processing system with standards-defined bus, such as a universal serial bus (USB), or the like, that permits external peripherals to be coupled to the apparatus 100. The transceiver 110 provides a means for communicating with various other apparatus over a transmission medium. The transceiver 110 may provide a proprietary wired interface or a wired interface compliant or consistent with a standard such as universal serial bus (USB), FireWire, Ethernet, Serial Advanced Technology Attachment (SATA), etc. The transceiver 110 may provide a wireless interface and transmit and receive radio signals through an antenna 116 using a proprietary or standardized signaling protocol such as IEEE 802.11, WiFi, WiMax, CDMA, WCDMA, Bluetooth, etc. The transceiver 110 and antenna 116 may enable the device to communicate as a radio frequency identification device (RFID) device. The transceiver may enable optical, infrared and other communications. Depending upon the nature of the apparatus, a user interface 112 (e.g., keypad, display, speaker, microphone, joystick) may also be provided.

The processor 104 is responsible for managing the bus 102 and general processing, including the execution of software stored on the computer-readable medium 106. The software, when executed by the processor 104, causes the processing system 114 to perform the various functions described infra for any particular apparatus. The computer-readable medium 106 may also be used for storing data that is manipulated by the processor 104 when executing software.

The various concepts presented throughout this disclosure may be implemented using a device that is configured to interface and/or interact with broad variety of telecommunication systems, network architectures, and communication standards.

In the example of electronic health records, portable computing devices may be used to authenticate a patient and/or a healthcare provider to enable and/or authorize and exchange of the electronic health records. The patient may elect to push electronic healthcare records to the healthcare provider. The healthcare provider may elect to push updates and/or new records to the patient. Healthcare records may include images, such as radiographic images initially captured through the use of radiography, magnetic resonance imaging (MRI), computerized tomography (CT-Scan or CATSCAN), ultrasonic imaging, or other imaging processes. Records and updates may be pushed over local networks using a Bluetooth connection, a wireless network or by optical exchange of information that provides a communication path that can be separate and distinct from the networking path used to deliver records. In one example, a quick response code (QRC) may be presented to a healthcare provider, whereby the QRC includes information that can be used to identify a network location of the records, cryptographic keys necessary to decrypt the records once retrieved from the network location, and other information.

The portable computing devices may directly deliver the portion of the information electronically using a Bluetooth connection, a wireless network or by an intermediate network server, or by any other method of electronic or wireless communication. Exchange of records and other information between the patient and the provider may be effected using multiple communications channels or links. In one example, a first channel may provide information that includes a network address of the records and corresponding cryptographic keys necessary to extract the records, while a second channel may be used to deliver the encrypted records and/or cryptographic keys. The first channel may be implemented using a camera or optical scanner to read an encoded optical image, such as a QRC or other barcode.

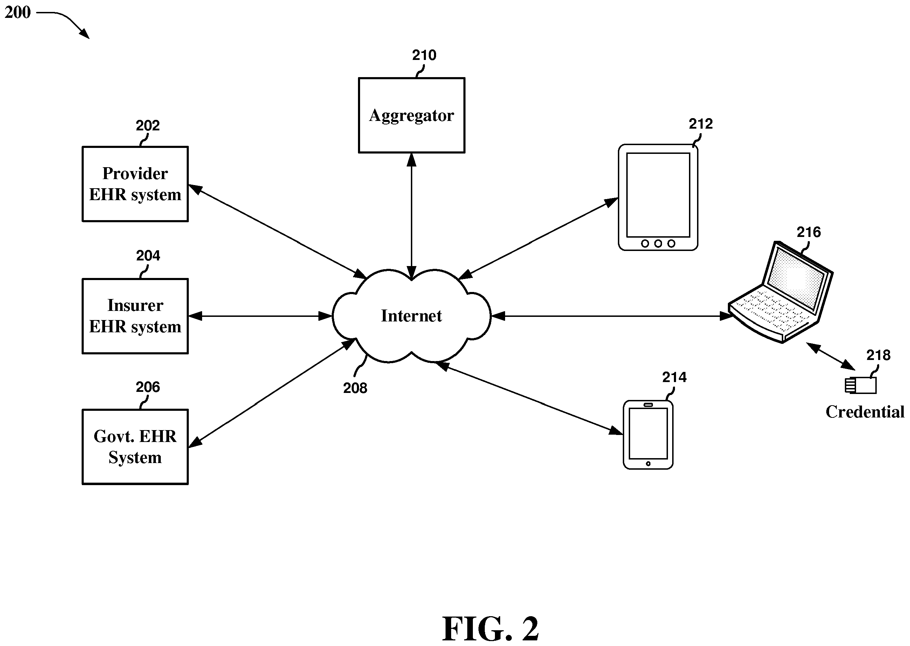

FIG. 2 illustrates a simplified example of a system 200 according to certain aspects of the invention. Electronic Health Records (EHRs) may be maintained in various physical locations and/or on systems 202, 204, and 206 operated by a plurality of different parties including healthcare providers 202, payors such as insurers 204 and/or government entities 206. In one example, records maintained on EHR systems 202, 204, and 206 may include duplicate information maintained in two or more of the EHR systems 202, 204, and 206. In other examples, that at least some EHR information may be aggregated, accumulated, and/or maintained in a single system 202, 204 or 206.

A user may access records through a mobile device 212 or 214, such as a smart phone, a tablet computing device, a notebook computer, or other suitable mobile device. In some instances, the user may access records through an appliance that incorporates or is controlled by a computing system or other processing device. The user is an individual record owner who may be a client or patient of a provider system and/or a client or an individual insured by an insurer, or an agent of the record owner. In certain circumstances, the user may be an emergency responder acting on behalf of a debilitated, injured or otherwise incapacitated individual record owner. In many instances, the record owner is a patient who receives healthcare services in multiple locations and/or from multiple healthcare providers. Healthcare providers may include one or more of a primary care provider (physician), a physician specialist, an emergency responder and a pharmacy. The patient may be insured by a private or public health insurance plan. Each of these different healthcare entities may maintain separate and distinct electronic health records for the patient.

The mobile device 212 or 214 may be adapted or configured, using an installed or downloaded application or agent to enable access to personal electronic health records that are maintained on one or more centralized databases corresponding to the EHR systems 202, 204 and 206. The user may access electronic health records related to a transaction or the provision of healthcare services to a patient, and the records accessed may comprise personal health records, such as medical records and insurance records, which may be remotely located on centralized databases embodied in EHR systems 202, 240, and 206 operated by a service provider, insurer or other entity.

In one example, databases maintained by one or more EHR systems 202, 204, and 206 may be accessed through a network 208. The network 208 may comprise one or more of a wireless network, a cellular access network, the Internet and/or a private network, etc. In certain embodiments, a record owner can access EHR systems or databases individually to retrieve records related to a specific activity, service, and/or provider. In some embodiments, the record owner may identify a set of EHR systems or databases to be accessed and combined, collated, or merged to obtain one or more of a combined record or combined report of EHRs. In some embodiments, the record user can specify a type of record to be accessed, regardless of which EHR systems or databases maintain such records. In some embodiments, a record owner can generate a combined individual record for immediate access and use by the user, or for delivery to a healthcare provider such as a physician, typically on the healthcare provider's own computing system 212. The record owner may produce a combined record on-demand (on-the-fly), or may provide access to a combined individual record that is maintained by, or on behalf of the record owner and which is typically updated automatically and/or periodically. In some embodiments, the record owner may authorize and/or enable a provider to access EHRs from a single source, from multiple sources, and/or from an aggregator 210. In some embodiments, a record owner may authorize and/or enable a provider to access certain types of records, regardless of the location of those records.

As illustrated in FIG. 2, the individual records may be delivered to a physician's mobile computing device 212, such as a tablet computer or smart phone, although the combined individual record may also be delivered to a server or other computer of an EHR system 202, 204 or 206. In some embodiments, the record owner may cause a server or other network device 210 to deliver the combined individual record to an EHR system 202, 204, or 206 and/or to a physician's mobile computing device 212 or other computing device, such as a desktop computer. In one example, the aggregator 210 may be used to provide individual records when a record owner does not have access to a device 214 capable of producing and delivering the individual record or when the record owner's device 214 cannot connect to provider's computing device 212 or systems 202, 204, or 206.

Identification and authentication information may be maintained on a record holder's device 214 to permit the record owner to access each of systems 202, 204, and 206. The maintenance and control of the identification and authentication information by the record owner can reduce overall system complexity because a single command and identification process at the record holder's device 214 can initiate automatic access to relevant records on the EHR systems 202, 204, 206 and/or to relevant records provided by an aggregator 210. For example, an agent installed on the record owner's mobile device 214 may be configured to identify and authenticate the user of the device 214 through password, challenge words, a biometric scan and/or other means for authentication known in the art. Authentication may optionally be confirmed by a trusted third party device or service provider. Authentication information may be provided to each of the EHR systems 202, 204, and 206 and/or the aggregator 210 to enable access to the EHR information related to the record owner.

The process of authentication and/or point of origin of the request may be recorded and may be used to prove consent of a record holder to a transfer of records to a provider. In some embodiments, a request from a user to transfer records may be considered to include consent of the record owner, based on prior identification and/or authentication of the identity of the user as the record holder. The record owner may be presented with a request to confirm a transfer request. The request for confirmation may include a request for identification and/or a request to authenticate the identity of the recipient of the transfer request. In some embodiments, the user may configure the type of transfer to be performed for each request. For example, consent may be limited to a subset of the owner's EHR record. In some embodiments, the record owner may configure a default specification of the types of record that can be transferred to one or more service providers. Authenticated requests to transfer information and acknowledgements of such requests, as well as acknowledgements of delivery and /or acceptance of a requested EHR may be logged at the user device 214, the physician device 212, a physician management system and/or one of the record holder systems 202, 204, 206 and/or 210.

The user may authorize and/or initiate an access to EHRs through a service provider facility. The user may prepare a combined EHR report or may store a set of EHR information from a variety of sources on a mobile device or on a storage device. Locally maintained information is typically encrypted. The record holder may transfer a portion or all of locally maintained information to a healthcare provider when seeking healthcare services. The user may also access certain records on-line from home to check on his insurance status, medical appointments, to see prescription refill status or to communicate by e-mail with his physicians.

Certain embodiments provide an interface to multiple electronic health records for both users and service providers. A user may provide authorization that enables a service provider to access some or all of the user's combined records. A first provider may, at the user's discretion, access the user's individual EHRs maintained by a second provider where the second provider may be physically located at a different healthcare facility. In one example, a physician may directly and easily access all of the user records necessary to obtain a current view of the user's complete medical history, insurance eligibility status, and other information. Moreover, medical practitioners can directly access the user's records in order to update the user's health information.

When transferring records, user identification information may be authenticated using any combination of a user ID, password, challenge question and biometric information. Typically, the transfer is made contingent upon a two-way identification of a record holder and a healthcare provider. In-person identification may be made using direct sight. Additionally, both parties' portable devices may establish a connection that is confirmed by both the record holder and the healthcare provider. In one example, the connection may comprise a session secured using encryption keys that are exchanged between the users. The encryption keys may be used to encrypt and decrypt information transmitted between the devices of the users. In some embodiments, the transfer may be restricted to proximately located devices. In one example, the record holder may initiate contact by selecting a physician's tablet computer from a list of devices within Bluetooth range, or within the same WiFi domain. The physician typically accepts the connection before the transfer is initiated.

In certain embodiments, records may not be exchanged without a positive identification of the recipient. When the record holder and the healthcare provider are located in different physical locations, information identifying a physical location may be provided by one or more of the record holder and the healthcare provider. The identification of a physical location may be made using a global positioning system, location information provided by a wireless network and from other sources, including triangulation by a cellular network. For example, certain wireless network telecommunications services can provide accurate positional information based on triangulation and/or certain signaling characteristics of mobile devices. In some embodiments, an authentication service may be used to verify identity of a record holder and a healthcare provider, and the record holder and the healthcare provider may be connected when the authentication service confirms identity of the parties, even when the parties are located in different physical locations.

In certain embodiments, user devices of a record holder and a healthcare provider may be incompatible and may not be capable of direct connection. For example, and Android-based device may not be able to connect securely with a tablet computer based on a different operating system. When incompatible devices are used, a gateway may be used to facilitate the connection of the devices and may provide extended handshake services that identify both devices and establish a secure link between the devices. The gateway may be provided using a local or network server and/or a cloud service.

In certain embodiments, global positioning technology may be used to confirm proximity or specific locations of the record holder and provider devices. In some embodiments, radio access technologies such as fourth generation long term evolution (4G LTE) may include location services that can be used to determine proximity or physical location information.

General purpose computing devices 216, such as a notebook or desktop computer, may also be used to access medical records, even where the computer 216 does not belong to the record owner. Record owner may provide an electronic credential 218 that, when read and used by computer 216, enables automatic access of combined individual records. Electronic credential 218 may comprise a hand-held device with a non-transitory memory and an embedded microprocessor or other programmable device. The electronic credentials may comprise a smart card, a USB flash drive, and radio-frequency identification (RFID) device, a Near Field Communication (NFC) token, web-enabled phones, etc. The electronic credentials may be embodied in an identification card or other format easily stored and secured by the user.

In certain embodiments, access to the user's EHR information may be obtained by presenting the electronic credential 218 to a computing device 212 or 216, whereby the computing device can establish a wired or wireless connection with the electronic credential 218 that enables an exchange of data. The electronic credential 218 may comprise a small portable device issued by an insurer, a government agency, a primary healthcare provider system, etc. The electronic credential 218 may comprise a memory that maintains information including a personal identifier, a unique identifier assigned to the individual, an EHR locator address, login information, and/or other identifying information. The user may use the electronic credential 218 to access one or more EHR systems 202, 204, and 206 through a computing device 212 or 216, such as a personal computer (PC), tablet computer, smart phone or other suitably equipped processing device. In one example, the electronic credential 218 comprises a flash drive, a smart card, or a device that can connect wirelessly to the computing device 212 or 216. The user may present the electronic credential 218 to the computing device 212 or 216 in a manner appropriate to allow the electronic credential 218 to exchange information with the computing device 212 or 216, whereby the computing device 212 or 216 may automatically access and login to one or more EHR systems 202, 204, and 206 using the record owner's identification. The user may have access to the EHR systems 202, 204, and 206 for automated and simultaneous real-time access to medical records maintained therein. In one example, an agent or other application software embedded in the electronic credential 218, or accessed through a network 208 using information stored on the electronic credential 218, may be downloaded to the computing device 212 or 216 to enable harvesting of selected data from the different EHR systems 202, 204, and 206 and generate an on-the-fly summary record for a physician to view and use.

Certain embodiments enable automated access to multiple data sources. In one example, an electronic credential 218 comprises an encrypted "electronic keychain" that may be maintained as a knowledge base that comprises identification and lists of sources of health related information for an individual. The knowledge base can include both the Internet address as well as identification and other credentials needed to enable access to the data. Typically the health information is maintained by a plurality of healthcare providers or practitioners, and information may be accessible through repositories or databases, including insurance databases and healthcare record portals.

An electronic credential 218 may comprise a device that includes a combination of hardware and software that can encrypt and decrypt information stored on the electronic credential 218. The electronic credential 218 may be embodied in intelligent electronic devices (devices having at least a programmable controller), such as a universal serial device, a smart phone, a PC and a tablet computer. The electronic device may have sufficient processing capacity and storage to operate as a self-contained EHR access portal.

In certain embodiments, an on-the-fly summary of health information can be provided at a medical provider facility, for example. Information provided by an electronic keychain may be used to initiate access and retrieval of information from multiple EHR sources 202, 204, and 206. Information provided by the electronic keychain may include one or more agents or applications that may compile multiple electronic health records into a single summary form. The summary form may be provided in a standardized format, such as continuity of care record ("CCR"), a continuity of care document ("CCD"), and other suitable formats. In some embodiments, compiled health records may be presented in a consistent summary format regardless of the format used by the originating source. Accordingly, information provided or accessed through the electronic keychain may include templates and conversion modules that can be used to filter and reformat EHR information from a variety of sources 202, 204, and 206.

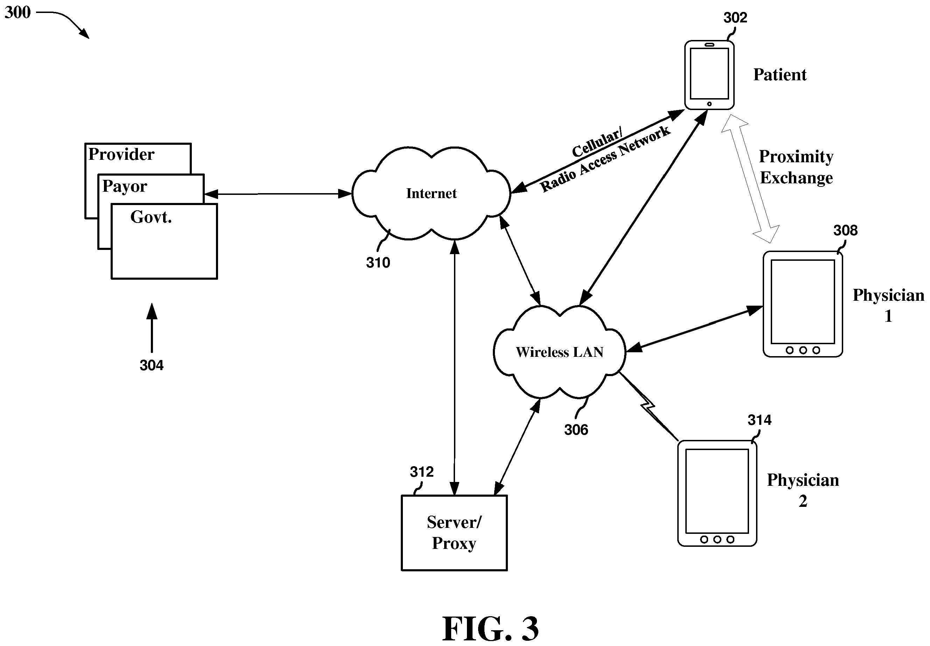

FIG. 3 is a block schematic 300 depicting an example of a network architecture that can support the various data flows involved in transactions related to the transfer of EHR records in accordance with certain aspects of the invention. In a first scenario, a record owner may use a personal portable computing device 302 to directly transfer, or push, a combined record to a first provider device 308. For example, a patient visiting a physician's office may wish to provide updated records to the attending physician. The patient may initiate an agent or other application on a smart phone 302 to perform the transfer. The user may be required to provide identifying information, such as a username, a password, an answer to a challenge question and/or the user may be required to provide biometric information. The user may typically select which records should be provided to the physician.

Upon authentication, the agent may determine if a single or combined record is maintained on the patient device 302 and whether such record is current. The agent may request records from one or more healthcare providers, insurers, government agency, public payor or other source of EHR information (shown generally at 304). Having combined or updated the individual record or records, the agent may cause the patient device 302 to push a single record or a set of combined records to the physician device 308 for immediate display. An application or agent on the physician device 308 may be manually initiated to receive the pushed information. In some embodiments, the physician device 308 may be adapted to respond to the push by opening an application or agent to receive or display the records upon receipt of a request for connection from patient device 302.

In certain embodiments, the physician may update records or retrieve other records on the physician device 308 and cause the updated or other records to be transmitted to the patient device 302. The patient device 302 may then provide the new or updated records to one or more of the EHR systems 304 or to another provider's computing device. In some embodiments, the physician may provide medical information to the patient device 302. For example, the physician may receive an X-Ray image on device 308 and may transfer the image to the patient device 302. In another example, the physician may cause device 308 to transmit information to the patient device that provides access to instructional or educational information to the patient device 302, including information on medications, dosage regimens and general information, such as educational information related to a medical condition.

The user device 302 and the physician device 308 may communicate using any available network or communication method, including WiFi, cellular communications, Bluetooth, IEEE 802.15 (Zigbee), and other short range wireless communications. In certain embodiments, communication between devices 302 and 308 may be restricted to the use of short range communications methods to enhance security. For example, the use of a Bluetooth link between physician device 308 and patient device 302 may limit communications range to a single room, allowing both the physician and patient to verify that communication is properly established between devices 302 and 308 and to ensure that the patient's privacy can be better protected. In certain embodiments, a patient may wish to transfer records to a physician who is not physically present using a wireless LAN 306 located in a medical facility and/or through the Internet 310 where the physician and patient are geographically remote from one another. In such cases, the patient and physician may establish a video conference connection to verify identities and to confirm that communication is properly established between the respective devices 302 and 308.

In a second scenario depicted in FIG. 3, a server 312 may act as an intermediary or proxy between patient device 302 and a second physician device 314. As described for the first scenario, the patient may initiate a records transfer using device 312. In certain embodiments, the intermediary server or proxy 312 may provide one or more services, including user identification and authentication services as well as record aggregation services when the patient device 302 is not configured or adaptable to perform such functions. For example, a record owner may provide an electronic credential 218 (see FIG. 2) to a general purpose computing device 216, whereby the electronic credential 218 causes the computer 216 to transmit a request for service to the proxy 312. In one example, the proxy 312 may provide a web page to the computing device 216 in order to permit the patient to initiate a request that may be executed by proxy 312 on behalf of the patient.

In another example, the patient device 302 and the second physician 314 may be unable to communicate directly. An intermediary 312 may be configured to perform a gateway or routing function that permits exchange of information between the respective devices 302 and 314 through a wide area network (such as the Internet) or a local area network, for example. The devices 302 and 314 may be unable to establish direct Bluetooth or WiFi connections with one another due to security settings of the second physician device 314 and/or the wireless LAN 306. In one example, the intermediary server or proxy 312 may provide a gateway function through the WiFi network 306 when the patient device 302 is connected to a different domain (e.g., a guest domain), while the second physician device 314 is connected via a secured private domain of the local network 306.

In certain embodiments, proximity may be defined as closeness in both place and time. A proximity exchange may occur when real-time communication of health records and/or health information occurs between patient and physician devices 302 and 308 while the devices 302 and 308 are in physical proximity with each other and the users can identify each other by direct sight. In certain embodiments, proximity exchange may be used to communicate health records and/or health information from a first mobile device 302 to a second mobile device 308 over a local wireless network during a specific time period. In certain embodiments, proximity exchange may be used to initiate the push of health records and/or health information to second mobile device 308 during a specific time period, whereby the proximity exchange is used for authentications and/or to provide information necessary for secure transmission of the health records and/or health information to the second mobile device 308.

The time period associated with a proximity exchange may be defined by a starting time when the communicating parties can identify each other by direct sight, either on a physical line-of-sight or by viewing each other through a video communication session. Typically, the two people exchanging information may be expected to be together in the same room during the proximity exchange. As an example, a patient with a mobile phone 302 can send his health records to his doctor who is waiting with his tablet 308 in the same examining room. In another example, the doctor at the end of the visit can send the patient treatment instructions or literature related to a diagnosis made by the doctor. In addition to having proximity of space (i.e. being in the same room) the patient and the doctor may also have proximity of time. Each party is expecting the communication to occur more or less immediately, for instance at the time when the physician is asking her patient about his medical history. In some embodiments, virtual identification can be made when the parties can see each other's face through a video link. In some instances, video link devices 302, 308, and 314 may be adapted to perform facial recognition, iris scanning, fingerprint scanning or other biometric scanning when direct and/or indirect visual identification cannot be made by the parties. In some embodiments, visual recognition or a biometric alternative is required to permit access to the EHR information to be exchanged between the parties.

Proximity exchange can provide improved security for EHR exchanges. Proximity exchanges typically limit an EHR exchange by location and time, and an EHR exchange may be initiated by an EHR owner in the presence of recipient of the EHR exchange. Moreover, the opportunity to complete an EHR exchange may be restricted in time, such that EHR exchange must be initiated within a predefined time. An EHR exchange may be characterized as a one-time push, whereby the push cannot be repeated and each push requires separate authorization by the record owner.

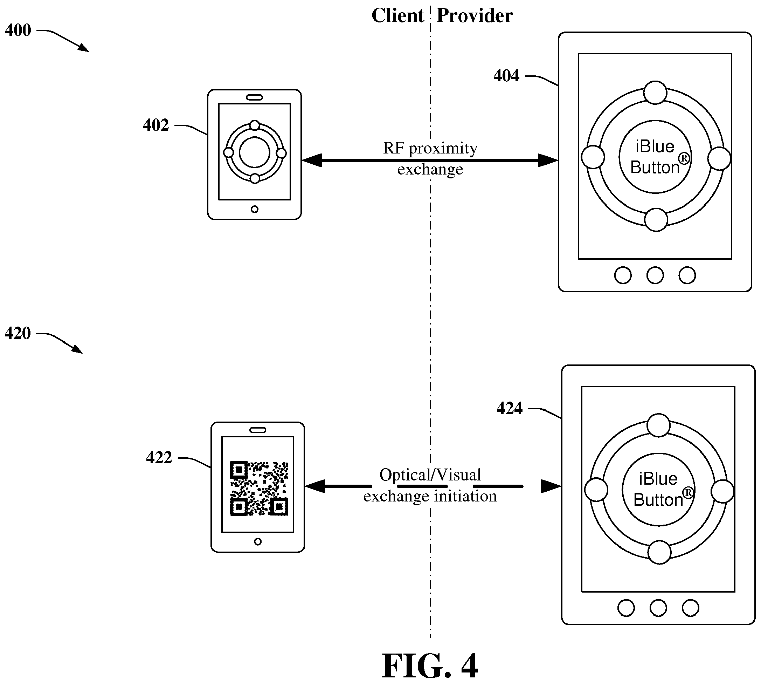

FIG. 4 includes examples 400 and 420 of proximity exchange that illustrate improved security in the example of an EHR exchange between a patient (client) and healthcare provider. Proximity exchange typically requires that both parties to the exchange are in the same location and/or can visually or audibly confirm the identity of the other party. Proximity exchanges also may employ limited range electronic communications, such as Bluetooth and other short range RF communications technologies, NFC interactions, RFID, optical communications, ad hoc connections, and so on. However, proximity exchange may also include exchanges that occur within the same building and/or wireless network segment or cell, when an affirmative identification of the parties can be made.

In one example 400, a proximity exchange is enabled when two devices 402, 404 and/or 422, 424 are in direct communication and proximately located. The client device 402 may be a smartphone, tablet, media player, appliance, or other suitable device. The client device 402 may be equipped with an agent or other downloaded application that is configured to provide access to EHR information associated with the client. The provider device 404 may be a personal computer, notebook, smartphone, tablet, media player, or other suitable device and may be equipped with an agent or downloaded application that provides provider access to one or more systems, including a practice management system, EHR systems 202, 204, 206, 210 (see FIG. 2), and other systems. The client, having decided to push EHR records to provider device 404, may interact with the agent or application on client device 402 to authenticate patient identity and initiate transfer. EHR exchange may be performed directly by client device 402, or indirectly through a proxy or other server. The client device 402 may transmit information wirelessly to the provider device 404, whereby the information may cause the agent or application on the provider device 404 to initiate receipt and acceptance of the EHR records. Typically, the client/patient may confirm that the push is targeting the provider device 404 based on a personal interaction with the provider and/or confirmation provided through interactions between the client device 402 and the provider device 404.

In another example 420, an EHR exchange can be secured even if client device 422 is not in communication with the provider device 424 through a networking connection. For example, both devices 422 and 424 may be independently connected to the Internet, but may be unable to connect by Bluetooth or by local networks such as a WiFi network, NFC or Zigbee. In some instances, the client and/or the provider may choose not to use wireless network authentication, or may be prohibited from using wireless network authentications. In some of these examples, secure EHR exchange may be provided through the use of an authentication process employing a wired network, and based on a proximate exchange of information.

In the depicted example 420, an EHR exchange may be secured by optically exchanging authentication information between two devices 422 and 424. The client device 422 may be a smartphone, tablet, media player, appliance or other suitable device that is equipped with a camera or optical reader. An agent or application installed on the client 422 provides access to EHR information associated with the client. The provider device 424 may be a personal computer, notebook, smartphone, tablet, media player, or other suitable device and may be equipped with a camera or optical reader. An agent or application installed on device 424 provides provider access to one or more systems, including a practice management system, EHR systems 202, 204, 206, 210 (see FIG. 2), and other systems.

The client, having decided to push EHR records to provider device 424, may interact with the agent or application on client device 422 to authenticate patient identity and initiate transfer. In order to authenticate the parties to the EHR exchange, the client device 422 may be configured to present an optical image on a display. The provider may capture the image through a camera integral to the provider device 424 or attached to the provider device 424. The image can be decoded to retrieve an encryption key, a file location, and/or other information necessary to authenticate the provider device 424 during the EHR exchange. The provider device 424 may be configured to generate and display an encoded image that can be captured by a camera of the client device 422 and decoded with a response or acknowledgement. In some embodiments, the exchange may be initiated at the provider device 424, which may create and display an image that is captured and used by client device 422 for identification purposes and to permit EHR records to be encrypted and/or directed to the provider device 424 during the EHR exchange. Any suitable type of encoded image may be used, including a barcode such as a QRC.

In certain embodiments, an EHR exchange may be secured by optically providing authentication information from a client device 502 to a provider device 504, without receipt of an express consent to the transaction by the client at the time the transaction occurs. Such exchange may occur, for example, between the client device 502 and a provider device 504 operated by a first responder paramedic, physician, nurse or other provider who is responding to an emergency. Accordingly, the mobile device 502 of an incapacitated client may provide authorization that enables a first responder or other provider to access client medical records without initiation of the transaction or transfer by the client.

In one example, the client device 502 may be configured to display, or provide access to a first-responder encoded image (FREI) on a home screen, login screen and/or other screen of the client device 502. In one example, the FREI may comprise an authentication QRC that can be displayed on a screen provided when a third party wishes to call an emergency service without logging onto the client device 502. In another example, an icon, link and/or reduced-size version of the FREI may be provided on a screen accessible by the first responder or other medical provider, such that activation of the icon, link and/or reduced-size FREI may display a full-size version of the FREI for scanning. In another example, first responders and other pre-authorized providers may enter information including a first-responder identification (FRID) at an initial logon screen of the client device 502 in order to access an authentication code, whereby the FRID may be universal to all client devices 502 subscribed to a wireless network system, and where the FRID may be changed on a regular basis. In some instances, the ID may be entered through a network, where the first responder device 504 initiates a call to the client device 502.

In certain embodiments, the FREI may be generated by the client and printed for use by first responders should an emergency occur. The printed FREI may be updated from time to time and may include sufficient information that provides a first responder with authorization to access the client's medical records using the provider mobile device 504. As described herein, the first responder may be required to provide identifying and authenticating information before access to the medical records is granted. The request sent to the server to fetch the client's medical records may contain provider mobile device 504 specific information, such as a unique device ID (UDID) on a tablet computer, for example. Accordingly, access to medical records may be restricted to pre-authorized devices based on identifying information of the devices.

The FREI may include information that identifies the client and provides access to some or all of the medical records of the client. Access may be limited to certain records which may be determined or expected to be relevant, necessary or desirable during an emergency involving the client. The client may provide advance authorization to permit access to the relevant medical records and the client may specify which records can be made available. In some instances, the client may provide graduated authorization that permits a first-responder access to detailed medical records necessary or useful for treating the client under foreseeable emergency conditions, and that permits public access to certain records or information that may be disclosed without compromising the client's privacy interests. An example of publicly accessible records may include "Medic-Alert" style information which identifies known medical conditions of the client that could render the client incapacitated, and/or that identify allergies suffered by the client, including drug allergies or resistance or reactions to drugs that could cause distress to the client if administered during an emergency situation.

In certain embodiments, the FREI may provide sufficient information that allows an authorized first responder or other provider to access client medical records subject to authentication of the identity of the first responder or provider. The first-responder may transmit a request that includes the FREI or information extracted from the FREI, together with identifying information that can prove the identity of the first responder and/or indicate levels of authorization to access medical records. In some instances, the first responder may be challenged by an authentication server or application to provide additional authenticating information. The first responder may be challenged if requests for certain types of client medical records are requested. Interactions with first responders and client medical records may be logged and cross-referenced to the first responder or other provider.

In one example of an embodiment, an application such as the iBlueButton® may be installed on the client device 502. The application may configure the client device 502 to provide a QRC on certain display screens of the client device 502, including the lock screen for example. A first responder or provider may scan the QRC using an iBlueButton Pro® application ("ProApp.") installed on a provider device 504 in order to facilitate transfer of the client medical records to the ProApp. during an emergency, even if the client is physically unable to authorize the transfer. The QRC may be visible when the client device 502 is not in active use. According to certain aspects of the invention, the QRC may be decoded only by authorized versions of the ProApp. In one example, the QRC may be decoded after an unlock code is entered into the ProApp. by a first responder. The QRC may be associated with a file transfer as disclosed herein. In some embodiments, downloaded medical records are not automatically deleted to ensure access by first responders and other providers responding to the emergency. In some embodiments, client records are deleted after their initial use in non-emergency situations.

In some embodiments, a first responder may identify a current medical condition of the client when requesting access to medical records. In practice, the request for medical records may be automated, such that the first responder may initiate an application or module on the provider device 504 in order to access medical records of the client. The application may be a customized emergency response application, and/or may comprise a provider application that includes an emergency procedure module. In some instances, the first responder may provide information related to the condition of the client and such information may be used to determine a subset of the client's medical records that can be provided to the first responder. The application may provide options and instructions that allow a first responder to operate the client device 502 in order to display the FREI for capture using the first responder's provider device 504.

In certain embodiments, the first responder's provider device 504 may automatically generate and transmit a request for medical records upon capture of the FREI. The request may be handled by one or more medical records as discussed herein, but using a preauthorization of the client to access necessary or useful records.

In certain embodiments, first responders and other medical providers may connect with an embedded computing system to gain access to EHRs belonging to an individual when called to provide assistance to the individual. The embedded computing system may be deployed in a vehicle or a household appliance, for example. The embedded computing system may be configured to maintain information related to one or more registered users or identified users of a device that includes the embedded computing system. In one example, an on-board vehicle management system, entertainment system, navigation system or other controller or appliance may be adapted to identify an occupant of an vehicle such as an automobile in order to provide customized service to the occupant. Identification may be made by manual selection, RFID such as an RFID embedded in a key or vehicle access device, biometric information captured by a system of the vehicle (e.g. a iris or fingerprint scan).

In one example, an occupant of a vehicle may be identified through detection of wireless devices operated by the occupant, where the wireless devices may be a mobile phone, media player, a tablet computer, a laptop computer, and so on. The presence of multiple occupants of a vehicle may be known, although not all occupants may be identifiable by a device or appliance of the vehicle. The identity of an occupant may be used to customize the cabin environment of the vehicle by adjusting seat positions, configuring an audio device, defining frequently used routes for a GPS navigation system, etc. This identity may be associated with emergency response procedures configured and authorized by the identified occupant in advance. Other type of embedded computing systems in other devices and appliances may perform customizations based on identity of persons present in the vicinity of the devices or appliances.

Devices and appliances may be adapted to maintain information that can provide access to EHRs of a current occupant of a vehicle or user of an embedded device or appliance. In one example, FRIDs may be maintained or associated with each potential user of a device or known occupants of the vehicle. The device or appliance may also be adapted to maintain authorizations to be used in case of an emergency. Emergency information including FRIDs, FRID associations and/or emergency authorizations may be provided to devices and appliances using a mobile computing device of a record holder. For example, a record holder may operate an application installed on a mobile computing device to transfer and configure the emergency information on the device or appliance. The application may be an iBlueButton® application, a configuration application provided by the vehicle manufacturer or supplier of a device or appliance. A device or appliance may visually or audibly greet a new device connected wirelessly or by wire and may invite a user of the new device to provide emergency response information. Typically, an owner of a vehicle, device or appliance may initiate a configuration process which offers an option to provide emergency information and to configure emergency response.

A first responder may automatically obtain authorization to access EHRs by interrogating a device or appliance and/or by responding to a communication initiated by the device or appliance. In one example, a first responder arriving at the scene of a traffic collision may obtain authorization to access EHRs of an injured occupant of a vehicle by providing an FRID to a device or appliance that maintains or has indicated it has access to emergency information of an occupant of the vehicle, and who may be the injured occupant. Upon validation of the FRID, the device or appliance may execute a proximity exchange such as one of the exchanges described in relation to FIGs. 3 and 4. Authorization to access the EHRs may be provided wirelessly and/or may involve transfer of information in a barcode displayed within the vehicle or on the device or appliance. In the example of a traffic collision, a vehicle may detect the collision and may provide emergency information through a remote diagnostics system such as systems operated by the OnStar™ Corporation. The information may then be forwarded for the use of first responders. Emergency information provided through vehicle monitoring systems may be encrypted such that only authorized third party responders may extract the encryption keys and identifiers necessary to access the EHRs of an injured occupant.

Emergency information maintained by a device or appliance may include some medical information that may be needed by a first responder even if access to EHRs is not sought. Such medical information may include information that identifies known medical conditions of the client that could render the client incapacitated, and/or that identify allergies suffered by the client, including drug allergies that could cause distress to the client if administered during an emergency situation, such as a traffic collision.

In some instances, automatically-initiated emergency authorizations to transfer EHRs may be rescinded by the owner of the EHRs. In one example, an occupant of a vehicle involved in a collision may be relatively uninjured and may respond to an alert of a device or appliance instructing the device or appliance that no transfer of EHRs should be performed. In another example, the uninjured occupant may block transfers of EHRs through an application (e.g. the iBlueButton® application) installed on a mobile computing device.

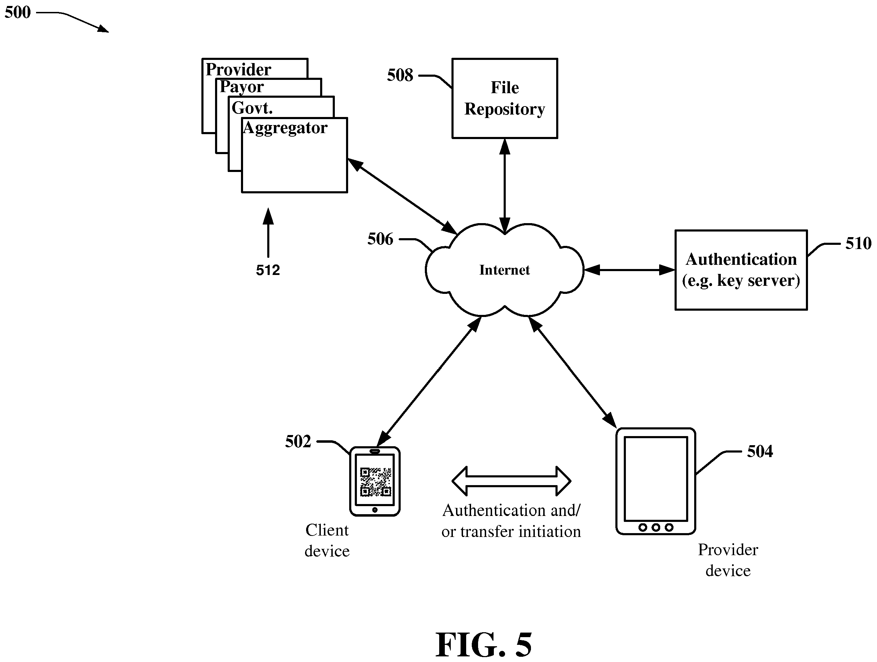

FIG. 5 is a block diagram illustrating a simplified example of a system that provides secured EHR exchange. Client device 502 may identify and/or prepare a set of EHR information for transfer to the provider device 504. For example, client device 502 may select EHR information from one or more sources to be transmitted to provider device 504. The EHR information may comprise records stored on client device 502. The EHR information may comprise records stored in one or more EHR systems and/or aggregators 512.

Client device 502 may then cause the selected EHR records to be stored in a file repository 508. File repository 508 may operate to provide a location for storage of a plurality of files and objects in a container that can be uniquely identified and accessed through a network such as the Internet 505. The container may be created for the duration of the EHR exchange and the container may be destroyed when the contents have been forwarded to the provider device 504, or after a predetermined time. File repositories may be implemented using an Internet cloud service such as Dropbox™ or Amazon S3™. The selected EHRs may be encrypted before being stored in the container.

In some embodiments, the client device 502 may provide information that enables access to the container in an encoded optical image that is displayed by client device 502. The information in the encoded optical image may include one or more of an address of the file repository 508, a name of the container that stores the EHRs selected by the client, an encryption key, and one or more usernames and passwords. The encoded optical image may be a QRC.

The provider may capture the encoded optical image and extract the location of the container and encryption keys need to decrypt the contents of the container. Typically, in-person acknowledgement is available in a proximity exchange, and the provider device 504 does typically provide an electronic message acknowledging capture of the optical image or even receipt of the EHRs to the client device 502. In at least some embodiments, electronic acknowledgement is made and such acknowledgements may be used for detailed logging of EHR exchanges by either the receiving or sending device. In some embodiments, the exchange of EHRs may be initiated by a provider and a patient may authorize transmission of EHRs to an address provided in an optical image displayed by provider device 504 and captured by client device 502.

In some embodiments, optical images may be transferred between devices 502 and 504 to enable direct communication of EHR records, to provide access to secured servers and/or to enable exchange of EHR information using encrypted Email or other communication systems. In some embodiments, optical images may be used to enable exchange of EHRs between parties connected by videoconference. For example, telemedicine may be employed to enable consultation between a physician specialist and a patient. Security for EHR exchange in such sessions may be augmented using encoded optical images captured from a videoconference display.

In certain embodiments, cryptographic keys may be exchanged by capturing an encoded image displayed on one or more of devices 502 or 504. An asymmetric key cryptographic process may be employed to improve security of the EHR exchange. Asymmetric key cryptography systems use two separate keys which are mathematically linked. The keys may be provided by an authentication service, which can generate public and private keys for the EHR exchange.

In certain embodiments, one or more logs may be configured to record the EHR exchange. When logging is required or preferred, components involved in the EHR exchange may provide affirmative acknowledgements of received information, including EHRs, content of EHR exchanges, authenticated user information, addresses of participants of EHR exchange, and/or date and time information corresponding to the EHR exchange. Logs may be maintained by the client device 502, provider device 504, EHR systems 512, repository 508 and/or a container management system associated with repository 508, and authentication service providers 510. Logs may be consolidated, formatted, summarized and/or aggregated by one or more of the client device 502, provider device 504, EHR systems 512, repository 508 and/or a container management system associated with repository 508, and authentication service providers 510. Typically at least one of the client device 502, provider device 504, EHR systems 512, repository 508 and/or a container management system associated with repository 508, and authentication service providers 510 maintains a log detailing one or more of a description of the EHRs stored in the container, or updated by the client or provider/recipient. Logs may also include information identifying the client, the recipient of the electronic healthcare records, and dates and times of transactions related to the electronic healthcare records stored in the container. Identification of members and providers may include member and/or provider numbers, biographic or demographic information as desired or permitted by regulatory authorities.

In certain embodiments, standardized health summaries can be made available to patients for easy download from government and private healthcare portals and to be shared with their healthcare providers. In some instances, immediate, proximate, secured exchange of health records and related health information is enabled between a patient and a physician or between two physicians. The exchange may be made in real time using mobile devices 302 and 308 (see FIG. 3). Certain embodiments of the invention enable secure and easy communication of EHR data from one mobile device 302 to another mobile device 308 over a local wireless network during a patient encounter with implicit or explicit patient consent. The exchange may take place in a physician's office, in an emergency room, an urgent care center, or at a hospital without a need to configure network servers and provider workstations with individual account names, addresses and security login parameters. A proximity exchange provides immediate access and secure exchange of individual health information at the time when the sender and the receiver of the information being exchanged can physically recognize each other and are reachable to each other over a network such as a wireless network.

In certain embodiments, a physician can exchange health information with a patient or with another physician using mobile devices 302, 308 and 314. The exchange can occur between two mobile phones, two tablet or other computers, or between a mobile phone and a tablet or other computer.

A patient device 302 may be adapted using an application or agent that securely stores and organizes personal health records and health information. The patient device 302 may be adapted using an application or agent that automatically accesses a patient portal account and can automatically login to retrieve current and updated patient health records. The patient device 302 may be further adapted to automatically download and combine health records from patient web portals using login and other identification and authentication maintained by the patient device 302.

In certain embodiments, the patient device 302 may be adapted to capture photographs of health documents and/or body parts using a camera in the mobile device 302. The patient device 302 may be adapted using an application or agent that accesses records created by other applications on the patient's mobile device. Proximity exchange may be used to transfer one or more health records and health information to a physician.

The patient device 302 may be adapted using an application or agent that directly receives health records, such as a visit summary, a referral note, test results, patient instructions, etc., from a physician using proximity exchange from the physician's mobile device 308.

The patient device 302 may be adapted using an application or agent that enables receipt of different types of records, including documents, photographs, audio and/or video recordings that may transferred by a physician using proximity exchange from the physician's mobile device 308 and the device 302 may be further configured to store and organize records exchanged to and from different physicians.

The physician device 308 may be adapted using an application or agent that can securely store and organize individual patient records and health information associated with several patients. The physician device 308 may be adapted using an application or agent that accesses records created by other applications, such as an electronic medical record (EMR) application, on the physician's mobile device 308.

The physician device 308 may be adapted using an application or agent that takes photographs of patient records and/or patient body parts using a camera of the mobile device 308. The physician device 308 may be further adapted to create an audio recording, including follow-up care instructions, and to store such recordings as part of the patient's record on the physician's mobile device 308.

The physician device 308 may be adapted using an application or agent that directly receives health records from a patient, using proximity exchange from the patient's mobile device and that downloads health related information from a variety of provider, electronic medical record, health information exchange and other portals.

In some embodiments, either the patient or the doctor can initiate a proximity exchange. The initiator of the communication may push a button or otherwise activate a function of an agent or application of their mobile device 302 or 308. The initiator device 302 or 308 may then broadcast over the wireless network an identification that may include a name that the other party can positively identify. The recipient may be notified that a request for proximity exchange has been received and may receive the name or names of the initiator. The recipient may choose between initiators detected within range of the recipient's mobile device 302 or 308 (e.g. a different physician and a different patient may be initiating an exchange in a nearby examining room). The proximity exchange may be authorized to commence when the recipient accepts the initiator.

In one example, Bluetooth and WiFi networks may be present. A mobile device may first attempt to advertise its desire to perform a proximity exchange using a WiFi Access Point (AP) if it is able to gain access to one within its wireless range. If the devices of both communication parties are able to access the same AP at the same time then the proximity exchange is performed through the AP, otherwise an attempt is made to connect them over Bluetooth. In some embodiments, Bluetooth connections are attempted first.

In certain embodiments, data is encrypted for transfer by proximity exchange. Encryption provides security that is not dependent upon on the security features of the underlying wireless network. Patient data such as health records and personal health information may be stored in encrypted form in mobile devices 302 and 308. In one example, encryption is performed using AES encryption algorithms with a secret encryption key that may be unique for the device 302 or 308. The encryption keys may be generated during configuration and installation of the agent or application on the device 302 or 308. Encryption keys may be based on a user password and a 64 byte random number. Encryption keys may be securely stored on the device in special secured hardware. This encryption protects both the confidentiality and the integrity of the data on the mobile devices 302 and 308.

Prior to transmission by proximity exchange, encrypted data may be first decrypted using the local cryptographic key of the sending device. The decrypted data may then be encrypted using a cryptographic key, which is known to both the sender and the receiver and which is created dynamically to exist only during the lifetime of the communication session. For example, the Diffie-Hellman algorithm may be used to create a communication session cryptographic key in such a way that only the two mobile devices 302 and 308 know the key. When encrypted data is received at the destination device 308 or 302, it can be decrypted using the key associated with current proximity exchange and then re-encrypted using the local cryptographic key of the destination device before it is stored.

In certain embodiments, health records and related health information can be securely exchanged in real-time without the need for predefined network infrastructure. Proximity exchange may provide secure communication between two parties who can physically recognize each other and can communicate electronically with each other over a network.

In certain embodiments, personal identification and contact information can be exchanged between patient device 302 and physician device 3080 as an option during proximity exchange. Personal identification information can include name, phone number, e-mail address, photograph, and such information may facilitate later contacts between the doctor and patient. In some embodiments, the contact information is exchanged automatically, without the requirement for each party to request it to be sent. Contact information may be automatically attached to records exchanged between the parties to enable easier filing and to enable accelerated retrieval on the respective devices 302 and 304.

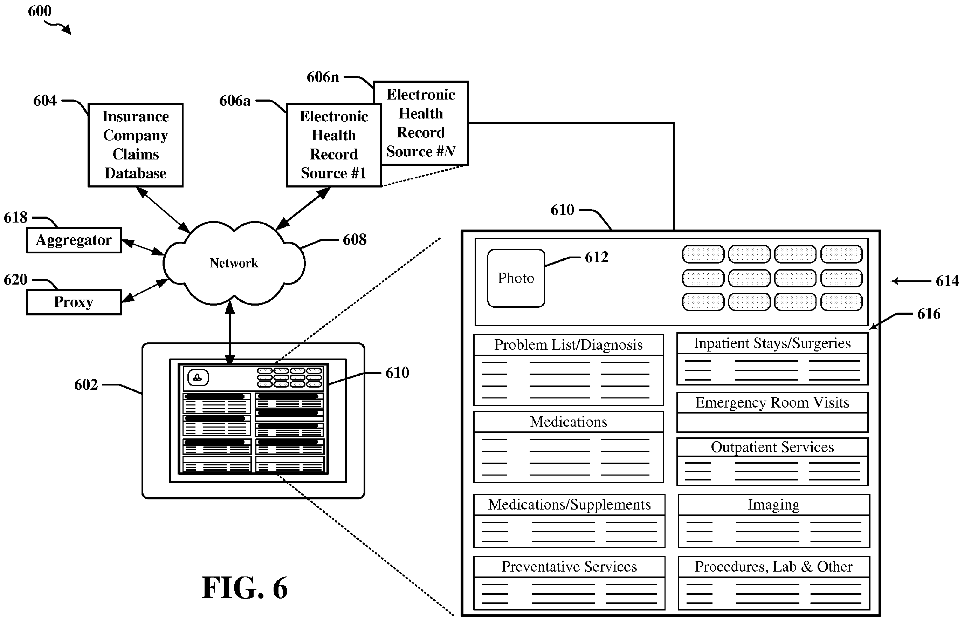

Record owners and providers may access the record owner EHR through a personalized portal provided on a mobile device or a conventional computing platform. Record owners may access their EHR information from a plurality of different sources and may provide one or more providers with partial or complete access to their EHR information. FIG. 6 illustrates a presentation of EHR information using a personalized portal according to certain aspects of the invention. The personalized portal may present a single display area that includes information from a plurality of sources including healthcare practitioners, insurance companies, an entity responsible for payment for services and other providers. EHR information may be combined remotely using a computer system or network server to access a plurality of EHR systems, before filtering and presenting the information to the record owner or provider. An aggregation server may reduce system complexity by providing identification, authentication, and qualification services related to the record owner and provider base as a centralized service, rather than requiring the plurality of EHR systems to maintain authentication information for the record owner and provider base. In some embodiments, a portal or agent may directly access and combine EHR information from the plurality of EHR systems.

Qualification services may filter results obtained from the plurality of EHR systems. Records received may be filtered based on certain predefined rules which may enforce government regulations. For example, certain records may not be accessible if access would cause healthcare information to be transferred between state or national jurisdictions. Records received may be filtered based on rules established by the record owner, a provider or the EHR system supplying the records. In one example, a record owner may determine a set of EHR records or a class of EHR records that should be withheld from one or more provider. The record owner may request that EHR records sent to a podiatrist should not include records related to psychiatric treatment, and vice versa.

An aggregator may format the information for display and/or may provide the information to an interface application that delivers a final format for display to the physician or other user. Interface application may be embodied in a portal or agent deployed on a record owner's computing device. Interface application may be provided as a plug-in on a network application at a provider location. Information provided by aggregator may be displayed in a web browser, a custom viewer application or in any suitable office automation application, such as a document reader or presentation tool. In certain embodiments, the display format may be specified and/or customized based on some combination of preferences and requirements of an end-user, a system administrator, a provider, payer and the record owner whose records are to be displayed. For example, the record owner may determine which fields are to be displayed and which data should be withheld. In another example, financial information is selected for display based on authorization levels set for the end-user.

In a certain embodiments, the record owner is a patient who receives, or expects to receive, healthcare services in a plurality of locations from multiple healthcare providers, such as his primary care provider (physician), a physician specialist and a pharmacy. The record owner may be insured by a private or public health insurance plan. Each provider may maintain separate and distinct electronic health records for the record owner. In some embodiments, record owner is permitted access to at least a portion of the records maintained by a provider on-line when such access is for the use of the record owner. For example, a record owner may access certain records from home to check on his insurance status, medical appointments, to view prescription refills, or communicate by e-mail with attending physicians.

Certain embodiments provide a record owner-controlled, practical, flexible, direct access to the record owner's health record that is continuously available. In some embodiments, the record owner may print and/or store a summary of online records on a removable storage device when it is necessary to present EHR records to one or more providers who are not users of the electronic delivery systems described herein. It will be appreciated however, that the printed or stored records are typically static and, if not updated in a timely manner, can become outdated by the time the records are presented at the point of care. Furthermore, the saved or printed record will typically not be available at all times, including during an emergency or at the time of a routine healthcare appointment, and may not be securely stored or carried; accordingly these stored or printed records can be subject to loss or tampering. Electronic access to EHR records may additionally resolve existing complex and ineffective patient consent management solutions, typically paper-based and single facility-based.

Consent may be provided by record owners as part of a request to deliver the record owner's EHR records. Certain embodiments provide direct access by healthcare providers to record owner records, whereby current record owner records are directly downloaded to the provider's system. The record owner may be required to provide authentication when requesting that a portion or all of the record owner's records are directly pushed to a provider system. In some embodiments, the record owner may also provide time-limited consent to permit a provider to request and access patient records directly from another service provider or from an aggregator. Consent may be provided directly by the record owner using a portal or agent, which may be implemented in a smart phone or other portable processing device.

A portal or agent may be provided on a computing device. A portal may provide access to a record owner's EHR information through a browser or an application or agent that resides temporarily on the computing device. The portal may comprise an application that is downloaded and executed through a browser or loaded from a portable storage device, such as a USB drive. In one example, a USB drive may be used as a credential to identify and/or authenticate a user of the USB drive, through encryption keys, biometric information, etc., and may provide an application that enables the record owner to establish a portal on the computing device. The USB drive or another credential may be issued by his insurer, the government, or his primary healthcare provider system, etc., and may maintain record owner information such as a personal and unique identifier assigned to the record owner, a record locator address and login. The USB drive may also be configured to maintain a previously downloaded EHR document, typically in encrypted form.

The portal may comprise one or more downloadable applications and may deliver services performed by a network server. An agent may be installed or otherwise maintained by a computing device. The agent typically performs one or more functions that allow a record owner to access EHR information. The agent may identify a wireless device such as an RFID, a Bluetooth-enabled device, a WiFi connected device or another device that can be used to identify the user. The agent may be an application installed on a smart phone, tablet computer or notebook computer, whereby the record owner may use an identifier to gain access to EHR information. Identification may comprise a combination of user ID, password, challenge, biometric information such as a fingerprint, iris scan, facial scan effected by an on-board camera, and so on.

The agent or portal may be configured to perform a plurality of functions including record owner identification and authentication, access to EHR records, identification and authorization of EHR records to be pushed to a provider, aggregation of EHR records and direct push of EHR records from the record owner's personal portal to a provider's system.

In certain embodiments, a record owner may use a smart portable device that has a processor and storage. The record owner may connect a flash drive, smart card, a wirelessly connectable storage device, or the like to the computer. In one example, the record owner may present an NFC device, such as an RFID or smart phone that responds to or activates an NFC receiver on a provider computing workstation. The record owner may also exchange authentication information with a provider using an optical reader or camera capture barcodes displayed by user or provider, and/or to capture biometric information that automatically enables access to the EHR information. Additionally, a device-to-device communication protocol between the patient's device and a provider's portable device may be employed to automatically access and exchange electronic health records, or initiate such exchange, with the healthcare provider.

FIG. 6 is a diagram 600 illustrating an example of delivery of EHR information to a computing device 602. The computing device 602 may be operated by a healthcare provider, and may comprise a tablet computer, a desktop computer, a notebook computer, or any other suitable computing device. The computing device 602 may receive and display a summary form 610 based on a patient's EHRs. The summary form is typically generated "on-the-fly" and/or on-demand. The summary form 610 may be dynamically updated to reflect activities in progress, or to add delayed information received from one or more sources of information 604, 606a-606n. The summary form 610 may be generated using information retrieved from local sources or through a network 608 which may include a local area network and/or wide area network such as the Internet. The summary form 610 may be generated from information retrieved from one or more EHR sources 606a-606n, insurance claims databases 604, or other sources. The summary form 610 may be generated from information provided by an aggregator 618 which combines information retrieved from one or more EHR sources 606a-606n, insurance claims databases 604, or other sources. The summary form 610 may be generated by an application provided in the computing device 602 or a proxy device or server 620.

The summary form 610 may be navigable, whereby a user of the computing device 602 may select certain items 616 in the summary form 610 to obtain more detailed information. The summary form 610 may include controls 614 that permit a user of the computing device to initiate actions. In one example, the controls 614 may include a button or button icon that, when activated, causes the computing device 602 to retrieve additional information including contact information of the patient, providers or payors. In another example, the controls 614 may include a button or button icon that, when activated, causes the computing device 602 to view additional information related to a patient history, including a family history, allergies, immunizations and/or implanted devices. In another example, the controls 614 may include a button or button icon that, when activated, causes the computing device 602 to export or print information from the summary form 610 or other information provided in the downloaded EHRs.

The summary form 610 may be tailored to the requirements of the user, whether an EHR holder, an insurance provider, a government agency, a physician or other healthcare provider. The summary form may be formatted for ease of viewing on any suitable platform. The summary form may be presented in a single view, window and/or screen to allow a physician or patient to access desired information in one place, with a minimum of required navigation. This single screen display can be generated on the fly and can include clinical information (e.g. in CCD/CCR format), administrative information and financial information, such as insurance eligibility information and past utilization and encounter information. The healthcare provider can typically obtain immediate access to the type, amount and location of services received by a patient, as well as out of pocket expenses incurred.

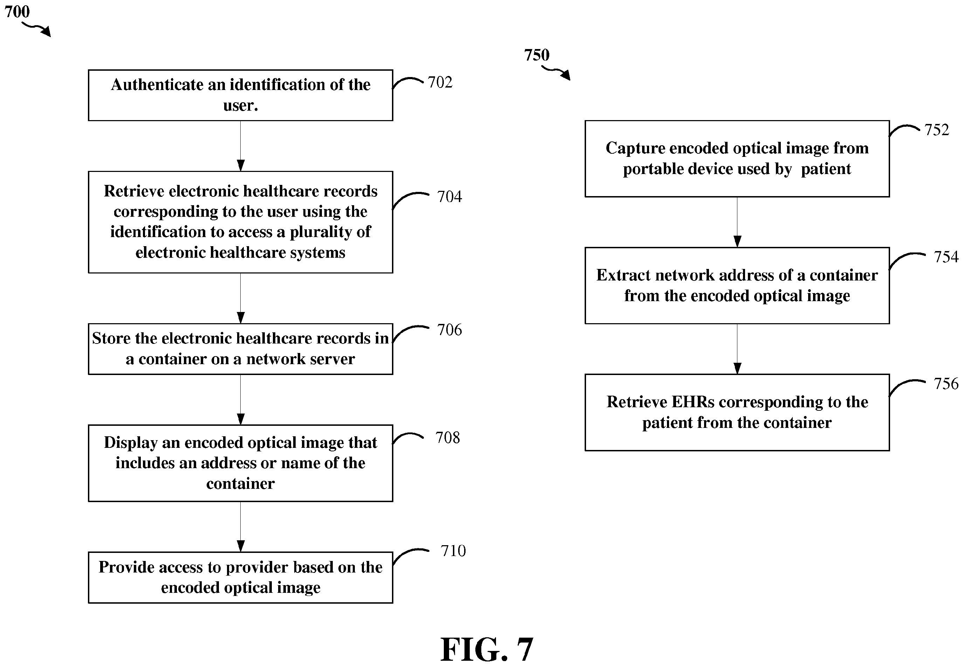

Certain processes according to certain aspects of the invention will now be described with reference to FIG. 7 and FIG. 2. For the purposes of the description, an example an embodiment of the invention used by military Veterans will be described, whereby a typical Veteran accesses healthcare at different Veterans Administration (VA) and non-VA provider sites and EHR information for the Veteran is maintained by government and non-government entities. In the example, an exchange can occur between points of care, whereby electronic health records, such as Blue Button records, can be automatically downloaded from various patient portals by a Veteran's portable computing device 214 or electronic credential 218, which has been adapted through the installation of an embedded application. Various patient portals may be accessed through mobile computing device 214, 216 and/or 218, the patient portals including "My Health*e*Vet" at the VA, TRICARE Online, and MyMedicare.gov, and other examples.

FIG. 7 includes a flowchart 700 that describes a method employing a records access system that may provide access to a provider to client records. In one example, the records comprise EHRs, the client may be a patient and the provider may be a healthcare provider.

At step 702, the client device 214 may authenticate an identification of the user.

At step 704, the client device 214 may retrieve electronic healthcare records corresponding to the user by using the identification to access a plurality of electronic healthcare systems.

At step 706, the client device 214 may store the EHRs in a container on a network server.

At step 708, the client device 214 may display an encoded optical image that includes an address or name of the container. The optical image may comprise a QRC, and/or another form of matrix code or barcode. The optical image may enable an intended recipient of the EHRs to retrieve the EHRs from the container. The EHRs stored in the container may be encrypted, and the encoded optical image may include one or more keys necessary to decrypt the EHRs retrieved from the container.

The optical image may be captured by a computing system used by the provider or the patient. The computing system may comprise a computer or mobile computing device that includes, or is coupled to, a camera or other optical sensor.

At step 710, the provider or the patient may access the EHRs in the container using information extracted from the optical image.

The client device 204 and/or the computing system used by the provider may comprise one or more of a wireless telephone, a smart phone and a tablet computer and wherein the portable computing device is configured to retrieve the information from the plurality of electronic healthcare records systems using a cellular wireless telephone network.

In some embodiments, the intended recipient of the electronic healthcare records receives the encoded optical image through a videoconference connection.

In some embodiments, the EHRs stored in the container may be deleted after a predetermined time or may be deleted after a first retrieval from the container.

In some embodiments, at least one of the portable computing device, the network server and a computing device associated with the recipient maintains a log detailing one or more of a description of the electronic healthcare records stored in the container, the identity of the user, information identifying an actual recipient of the electronic healthcare records, and dates and times of transactions related to the electronic healthcare records stored in the container.

Veteran patient may present an ID card 218 that comprises a USB flash drive. The ID card may enable automatic communication/exchange of online health records with a provider EHR system 202. At step 704, software embedded in the Veteran's card 218 is automatically loaded and executed upon insertion and/or detection by an Internet-ready computing device 216. Typically, no software or system integration is requires and the software may directly launch a login screen for entry of the Veteran's single chosen password in order to grant the provider consent of the patient to proceed.

At step 706, the device embedded software may then auto launch and automatically login into one or more of the Veteran's selected EHR enabled patient portals. The computing device 216 may then download and combine EHR records, automatically and as directed by the device embedded software. The device embedded software may additionally reformat the downloaded EHR information into a clinically prioritized format in a single view (see 402). This single view may also include a reply prompt window for the provider to send, at step 710, a follow up note, with or without attachments, to the Veteran's primary care or referring physician. The follow up note may be transmitted by secure Email, Fax and/or secure messaging.

As shown in Fig. 2, a Veteran's mobile device 214 may comprise a smart phone or tablet computer on which an application or agent has been installed or embedded. The application or agent may adapt the Veteran's device 214 to maintain at least a summary report of EHR records on the device. The application or agent may also adapt the Veteran's device 214 to automatically access one or more EHR portals and store the EHR records in a container, or receive EHR records via the Direct protocol. In some embodiments, records can be pushed to the provider device upon consent and authentication of the Veteran. The records may be pushed to a provider device 212 using, for example, a service discovery protocol. An application or agent on the provider device 212 may signal its presence, which enables the Veteran to execute a transfer of records by commanding device 214 to directly push selected records to the provider's device 212. The provider may be prompted to choose whether or not to accept the Veteran's records before or after transmission of the records by the Veteran's device 214.

The physician may optionally provide updates records to Veteran's device 212, 214 or 218 which may then be relayed to the EHR systems 202, 204, or 206 through one or more portals. Typically, the provider reviews the received records and is provided a reply prompt to send information to the Veteran's device 214. For example, the information sent by the physician may include a follow up note to the Veteran's primary care or referring physician. Optionally information such as a follow-up note may be transmitted by secure Email, Fax and/or secure messaging.

FIG. 7 also includes a flowchart 750 that describes a method employing a records access system that may provide access to a provider to patient records. In one example, the records comprise EHRs, the client may be a patient and the provider may be a healthcare provider.

At step 752, a computing device associated with a provider of healthcare services may capture an encoded optical image from a portable computing device presented by a patient. The encoded optical image may comprise a QRC or other barcode.

At step 754, the computing device associated with a provider of healthcare services may extract an address of a container from the encoded optical image. The container may be located on a network server. EHRs may be stored in the container. The EHRs stored in the container may be encrypted. The encoded optical image may include one or more keys necessary to decrypt the electronic healthcare records stored in the container.

At step 756, the computing device associated with a provider of healthcare services may retrieve electronic healthcare records corresponding to the patient from the container. The EHRs stored in the container may be deleted after a predetermined time, and/or after a first retrieval.

The computing device associated with the provider may comprise one or more of a wireless telephone, a smart phone and a tablet computer. The computing device associated with the provider may be proximately located with the portable computing device. In some embodiments the computing device associated with the provider may be remote with respect to the portable computing device, and the encoded optical image may be received through a videoconference connection.

In some embodiments one or more components of the system may maintain a log of transactions associated with the user and/or the EHRs. At least one of the portable computing device, the network server and the computing device associated with the provider may maintains a log that details one or more of a description of the electronic healthcare records provided in the container, the identity of the patient, information identifying the provider times of transactions related to the electronic healthcare records stored in the container.

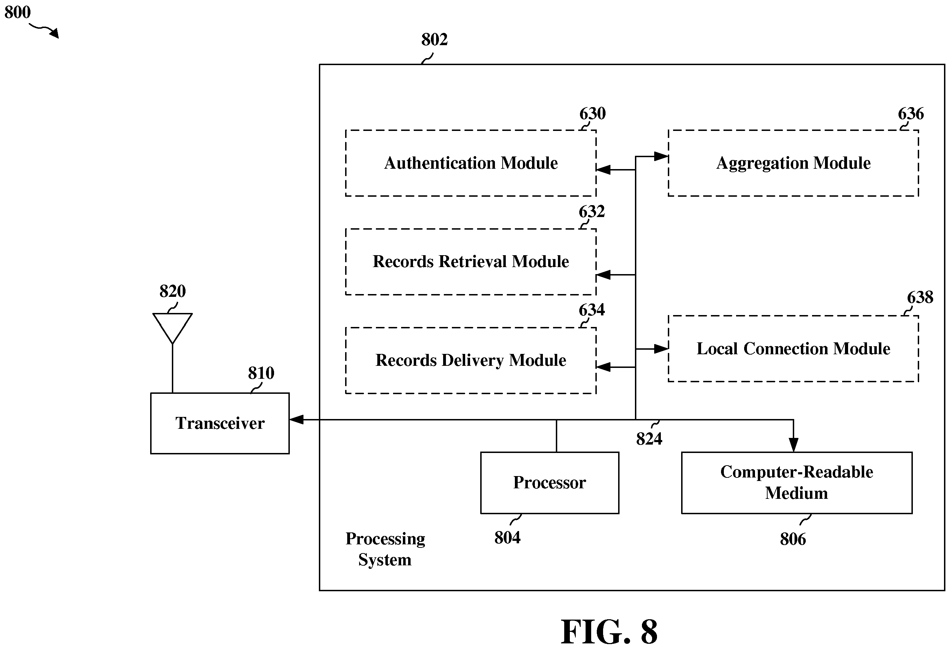

FIG. 8 is a conceptual block diagram 800 illustrating the functionality of an exemplary apparatus 802 as used in a provider location for accessing medical records. The apparatus 800 may be a portable or non-portable computing device, having a processor 804 and non-transitory storage 806 in which an agent or software may be installed that includes one or more modules 830, 832, 834, 836 and 838.

The apparatus 800 may include an authentication module 830 identifies and/or authenticates the user associated with the apparatus 800. Module 830 may identify the user using a biometric measurement, a password, user identifier, RFID device and/or a challenge.

The apparatus 800 may include a records retrieval module 832 that automatically retrieves information corresponding to the one user from at least one electronic healthcare records system using the identification to access the at least one electronic healthcare records system. The apparatus 800 may retrieve the information from the at least one electronic healthcare records system using a cellular wireless telephone network.

The apparatus 800 may include a records delivery module 834 that electronically delivers a portion of the information to a healthcare provider. The apparatus may deliver the information using transceiver 810 and antenna 820, which may be configured to support Bluetooth communications and/or communications through a wireless network, such as a WLAN or cellular network. Accordingly, the apparatus 800 may comprise one or more of a wireless telephone, a smart phone and a tablet computer. A portion of the information may be delivered to a different computing device operated by the healthcare provider. A portion of the information is delivered using a server communicatively coupled to the portable computing devices associated with the one user and operated by the healthcare provider. A portion of the information may be encrypted.

The apparatus 800 may include a local connection module 838 that establishes a data and/or audio-visual link with a provider. The apparatus 800 may establish a connection using transceiver 810 and antenna 820, which may be configured to support Bluetooth communications and/or communications through a wireless network, such as a WLAN or cellular network. Accordingly, the apparatus 800 may comprise one or more of a wireless telephone, a smart phone and a tablet computer. Module 838 may perform other functions, including automatically providing consent to allow providers to download records or the user.

The apparatus 800 may include an aggregation module 836 that combines the retrieved information with other information retrieved from the at least one electronic healthcare records system to obtain combined information. The other information may comprise electronic health records of the user that are maintained by the apparatus 800. Electronic health records maintained by the apparatus may be encrypted using encryption keys uniquely associated with the one user.

One or more of modules 830, 832, 834, 836 and 838 may combine to perform a method comprising the steps of receiving from a first portable computing device, information identifying a user of the first portable computing device and a request for selected healthcare records corresponding to the user and an identity of a healthcare provider, causing the first portable computing device to authenticate identity of the user, wherein the authentication of the identity of the user serves as a consent of the user to release the selected healthcare records, and upon receiving information confirming the authentication of the identity of the user, transferring the selected healthcare records to a second computing device operated by the healthcare provider. In some embodiments the portable computing device maintains encrypted information that identifies the user.

The method may further comprise updating at least a portion of the selected healthcare records using information received from the healthcare provider. The method may further comprise healthcare records other than the selected healthcare records using information received from the healthcare provider. The method may further comprise creating new healthcare records using information received from the healthcare provider.

In some embodiments, the selected healthcare records comprise records from a plurality of sources, including at least one provider source and a payer source. In some embodiments, transferring the selected healthcare records includes receiving an acceptance from the healthcare provider. In some embodiments, the user and the healthcare provider are located in close proximity and wherein the transferring the selected healthcare records is contingent on a direct visual identification made by one or more of the user and the healthcare provider. In some embodiments, the user and the healthcare provider are located in different rooms and wherein the transferring the selected healthcare records is contingent on a virtual visual identification made by one or more of the user and the healthcare provider.

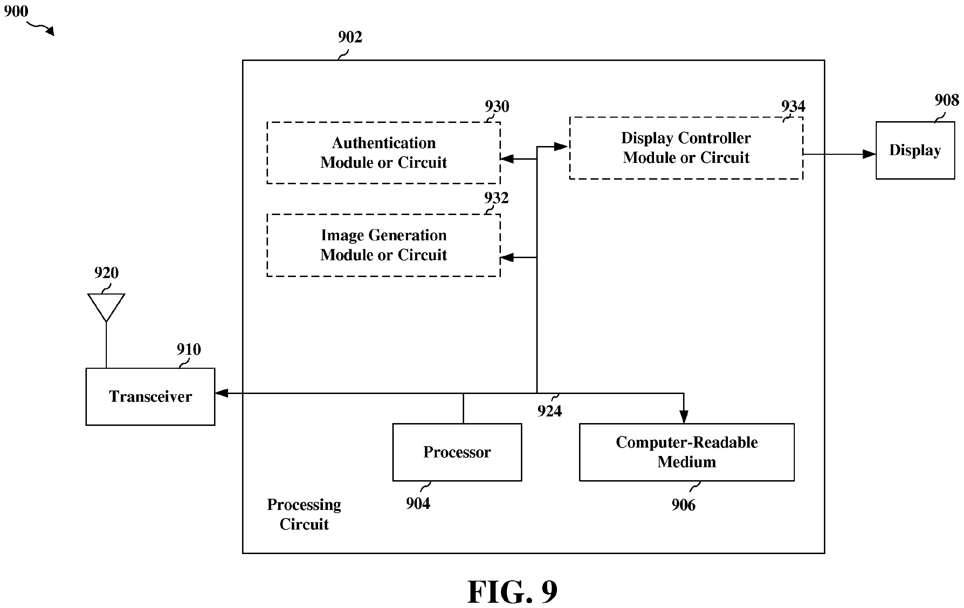

FIG. 9 is a diagram 900 illustrating a simplified example of a hardware implementation for an apparatus employing a processing circuit 902. The processing circuit 902 typically has a processor 904 that may include one or more of a microprocessor, microcontroller, digital signal processor, a sequencer or a state machine. The processing circuit 902 may be implemented with a bus architecture, represented generally by the bus 924. The bus 924 may include any number of interconnecting buses and bridges depending on the specific application of the processing circuit 902 and the overall design constraints. The bus 924 may interconnect various circuits including processors and/or hardware modules, represented by the processor 904, the modules or circuits 930, 932 and 936, a transceiver 910 configurable to communicate wirelessly an antenna 920 and the computer-readable storage medium 906. The bus 924 may also link various other circuits such as timing sources, peripherals, voltage regulators, and power management circuits, which are well known in the art, and therefore, will not be described any further.

The processor 904 may be responsible for general processing, including the execution of software stored on the computer-readable storage medium 906. The software, when executed by the processor 904, may cause the processing circuit 902 to perform certain of the functions described *supra* for any particular apparatus. The computer-readable storage medium 906 may also be used for storing data that is manipulated by the processor 904 when executing software, including data encoded in images and symbols transmitted wirelessly. The processing circuit 902 further includes at least one of the modules 930, 932 and 934. The modules 930, 932 and 934 may be software modules running in the processor 904, resident/stored in the computer readable storage medium 906, one or more hardware modules coupled to the processor 904, or some combination thereof. The modules 930, 932 and 934 may include microcontroller instructions, state machine configuration parameters, or some combination thereof.

In one configuration, the apparatus 900 includes a module and/or circuit 930 that is configured to authenticate an identification of a user of a mobile device, a module and/or circuit 934 or 910 that is configured to communicate an electronic authorization from the mobile device to a provider device using a first communication method. The electronic authorization may enable the provider device to have access to electronic healthcare records of the user. The access to the electronic healthcare records of the user may be provided through a second communication method that is different from the first communication method. In one example, the first communication method is initiated by the mobile device after the user of the mobile device has been authenticated, and comprises transferring an image between the mobile device and the provider device. The image may be generated by the module and/or circuit 932 that may be configured to encode information identifying the user of the mobile device, and an address of the electronic healthcare records of the user. A module and/or circuit 934 may be is configured to display the image using the display 908. The image may be captured from the display 908 by a camera of the provider device. The display may be provided as an internal or integral component of the apparatus 900, or the processing circuit 902. The display 908 may comprise an external display system, such as a videoconferencing display that is controlled or operated through the processing circuit 902.

In one example, the apparatus 900 may comprise a mobile device, which may be configured to authenticate an identification of a user of a mobile device 900 and communicate communicating an electronic authorization from the mobile device to a provider device using a first communication channel. The electronic authorization may enable the provider device to access EHRs of the user. Access to the electronic healthcare records of the user may be provided through a second communication channel that is different from the first communication channel. The first communication channel may be used by the mobile device to transfer an image between the mobile device and the provider device after the user of the mobile device has been authenticated. The image may be displayed by the mobile device for capture by a camera of the provider device. The image may include encoded information identifying the user of the mobile device. The image may include an address of the electronic healthcare records of the user. The image may include cryptographic keys.

The image may be displayed by the mobile device for capture by a camera of the provider device. The provider device may be configured to use the cryptographic keys to access the electronic healthcare records of the user. The image may include a QRC or a barcode.

The first communication channel may include a video link through a network connecting the mobile device and the provider device. The first communication channel may be a network controlled by a Near Field Communications protocol, a Bluetooth protocol or a Zigbee protocol. The second communication channel may include a wide area network that is configured to provide access to a container on a network server. The EHRs of the user may be encrypted. The EHRs of the user may be deposited in the container. The EHRs of the user deposited in the container may be deleted after a predetermined time. The EHRs of the user deposited in the container may be deleted after a first retrieval of the electronic healthcare records of the user from the container.

At least one of the mobile device, the provider device and the network server may maintain a log related to transactions involving the container. The log may record a description of the EHRs deposited in the container. The log may record the identity of the user of the mobile device. The log may record an identity of the provider device when the provider device accesses the container.

## Claims

1. A method for controlling access to electronic medical records, stored in a container on a network server, the method comprising:
authenticating (702) an identification of a user of a mobile device (402, 422), the user being an owner of the electronic medical records, and the mobile device (402, 422) being a first mobile computing device (402, 422); and
communicating an electronic authorization from the mobile device (402, 422) to a provider device (404, 424) using a first communication channel;
wherein the electronic authorization provides the provider device (404, 424) with access to electronic healthcare records of the user;
wherein the access to the electronic healthcare records of the user is provided through a second communication channel that is different from the first communication channel; and
wherein the second communication channel comprises a wide area network that is configured to provide access to the container on the network server, wherein the electronic healthcare records of the user are encrypted and deposited in the container, and the electronic healthcare records deposited in the container are deleted after a first retrieval of the electronic healthcare records of the user from the container.

2. The method of claim 1, wherein communicating an electronic authorization from the mobile device (402, 422) to a provider device includes:
transferring an encoded image from the mobile device (402, 422) to the provider device (404, 424) after the identification of the user of the mobile device (402, 422) has been authenticated.

3. The method of claim 2, wherein transferring the encoded image from the mobile device (402, 422) to the provider device (404, 424) includes:
displaying (708) the encoded image on a display of the mobile device (402, 422), wherein the encoded image provides information identifying the user of the mobile device (402, 422) and an address of the electronic healthcare records of the user when the encoded image is captured (752) by a camera of the provider device and decoded by the provider device (404, 424).

4. The method of claim 3, wherein the provider device (404, 424) is configured to use cryptographic keys provided in the encoded image to access (756) the electronic healthcare records of the user.

5. The method of claim 2, wherein the first communication channel comprises a video link through a network connecting the mobile device (402, 422) and the provider device.

6. The method of claim 1, wherein the first communication channel is controlled by a Near Field Communications protocol, a Bluetooth protocol or a Zigbee protocol.

7. The method of claim 1, wherein the electronic healthcare records deposited in the container are deleted after a predetermined time if not retrieved from the container.

8. The method of claim 7, wherein at least one of the mobile device (402, 422), the provider device (404, 424) and the network server maintains a log related to transactions involving the container, and wherein the log records one or more of a description of the electronic healthcare records deposited in the container, the identity of the user of the mobile device (402, 422), or an identity of the provider device (404, 424) when the provider device (404, 424) accesses the container.

9. A system comprising a mobile computing device (402, 422) and a network server, wherein the mobile computing device (402, 422) is configured for wireless communications and comprises:
a processing circuit, wherein the processing circuit is configured to:
authenticate an identification of a user of the mobile computing device (402, 422); and
communicate an electronic authorization from the mobile computing device (402, 422) to a provider device (404, 424) using a first communication channel;
wherein the electronic authorization provides the provider device (404, 424) with access to electronic healthcare records stored in a container on the network server owned by the user;
wherein the access to the electronic healthcare records of the user is provided through a second communication channel that is different from the first communication channel;
wherein the second communication channel comprises a wide area network that is configured to provide access to the container on the network server;
wherein the electronic healthcare records of the user are encrypted and deposited in the container; and
wherein the electronic healthcare records deposited in the container are deleted by the network server after a first retrieval of the electronic healthcare records of the user from the container.

10. The system of claim 9, wherein the processing circuit is configured to communicate an electronic authorization from the mobile computing device (402, 422) to a provider device by:
causing an encoded image to be displayed on a display of the mobile computing device (402, 422), wherein the encoded image provides information identifying the user of the mobile device (402, 422) and an address of the electronic healthcare records of the user when the encoded image is captured by a camera of the provider device (404, 424) and decoded by the provider device (404, 424).

11. The system of claim 10, wherein the encoded image comprises a quick response code or a barcode and includes cryptographic keys and the image is displayed by the mobile computing device (402, 422) for capture by a camera of the provider device (404, 424), and wherein the provider device (404, 424) is configured to use the cryptographic keys to access the electronic healthcare records of the user.

12. The system of claim 9, wherein the electronic authorization is communicated using a Near Field Communications protocol, a Bluetooth protocol or a Zigbee protocol.

13. The system of claim 9, wherein the electronic healthcare records deposited in the container are deleted after a predetermined time if not retrieved from the container.

14. The system of claim 13, wherein at least one of the mobile computing device (402, 422), the provider device (404, 424) and the network server maintains a log related to transactions involving the container, and wherein the log records one or more of a description of the electronic healthcare records deposited in the container, the identity of the user of the mobile computing device (402, 422), or an identity of the provider device when the provider device (404, 424) accesses the container.

## Patentansprüche

1. Verfahren zum Kontrollieren des Zugriffs auf elektronische Patientenakten, die in einem Container auf einem Netzwerkserver gespeichert sind, wobei das Verfahren
Folgendes umfasst:
Authentifizieren (702) einer Identifizierung eines Benutzers einer mobilen Vorrichtung (402, 422), wobei der Benutzer ein Eigentümer der elektronischen Patientenakten ist und die mobile Vorrichtung (402, 422) eine erste mobile Rechenvorrichtung (402, 422) ist; und
Übermitteln einer elektronischen Autorisierung von der mobilen Vorrichtung (402, 422) an eine Bereitstellungsvorrichtung (404, 424) unter Verwendung eines ersten Kommunikationskanals;
wobei die elektronische Autorisierung der Bereitstellungsvorrichtung (404, 424) Zugriff auf elektronische Gesundheitsakten des Benutzers bereitstellt;
wobei der Zugriff auf die elektronischen Gesundheitsakten des Benutzers über einen zweiten Kommunikationskanal bereitgestellt wird, der sich vom ersten Kommunikationskanal unterscheidet; und
wobei der zweite Kommunikationskanal ein Weitverkehrsnetzwerk umfasst, das konfiguriert ist, um Zugriff auf den Container auf dem Netzwerkserver bereitzustellen, wobei die
elektronischen Gesundheitsakten des Benutzers verschlüsselt und in dem Container abgelegt werden, und die in dem Container abgelegten elektronischen Gesundheitsakten nach einer ersten Datenrückgewinnung der elektronischen Gesundheitsakten des Benutzers aus dem Container gelöscht werden.

2. Verfahren nach Anspruch 1, wobei das Übermitteln einer elektronischen Autorisierung von der mobilen Vorrichtung (402, 422) an eine Bereitstellungsvorrichtung Folgendes umfasst:
Übertragen eines codierten Bildes von der mobilen Vorrichtung (402, 422) an die Bereitstellungsvorrichtung (404, 424), nachdem die Identifizierung des Benutzers der mobilen Vorrichtung (402, 422) authentifiziert worden ist.

3. Verfahren nach Anspruch 2, wobei das Übertragen des codierten Bildes von der mobilen Vorrichtung (402, 422) an die Bereitstellungsvorrichtung (404, 424) Folgendes umfasst:
Anzeigen (708) des codierten Bildes auf einer Anzeige der mobilen Vorrichtung (402, 422), wobei das codierte Bild Informationen bereitstellt, die den Benutzer der mobilen Vorrichtung (402, 422) und eine Adresse der elektronischen Gesundheitsakten des Benutzers identifizieren, wenn das codierte Bild durch eine Kamera der Bereitstellungsvorrichtung erfasst (752) und durch die Bereitstellungsvorrichtung (404, 424) decodiert wird.

4. Verfahren nach Anspruch 3, wobei die Bereitstellungsvorrichtung (404, 424) konfiguriert ist, um kryptografische Schlüssel zu verwenden, die in dem codierten Bild bereitgestellt sind, um auf die elektronischen Gesundheitsakten des Benutzers zuzugreifen (756).

5. Verfahren nach Anspruch 2, wobei der erste Kommunikationskanal eine Videoverbindung über ein Netzwerk umfasst, das die mobile Vorrichtung (402, 422) und die Bereitstellungsvorrichtung verbindet.

6. Verfahren nach Anspruch 1, wobei der erste Kommunikationskanal durch ein Nahfeldkommunikationsprotokoll, ein Bluetooth-Protokoll oder ein Zigbee-Protokoll gesteuert wird.

7. Verfahren nach Anspruch 1, wobei die in dem Container abgelegten elektronischen Gesundheitsakten nach einer vorbestimmten Zeit gelöscht werden, falls sie nicht aus dem Container rückgewonnen werden.

8. Verfahren nach Anspruch 7, wobei mindestens eine/r der mobilen Vorrichtung (402, 422), der Bereitstellungsvorrichtung (404, 424) und des Netzwerkservers ein Protokoll führt, das sich auf Transaktionen bezieht, die den Container einschließen, und wobei die Protokollakten eine oder mehrere einer Beschreibung der in dem Container abgelegten elektronischen Gesundheitsakten, der Identität des Benutzers des mobilen Vorrichtung (402, 422) oder einer Identität der Bereitstellungsvorrichtung (404, 424) aufzeichnet, wenn die Bereitstellungsvorrichtung (404, 424) auf den Container zugreift.

9. System, umfassend eine mobile Rechenvorrichtung (402, 422) und einen Netzwerkserver, wobei die mobile Rechenvorrichtung (402, 422) für drahtlose Kommunikation konfiguriert ist und Folgendes umfasst:
eine Verarbeitungsschaltung, wobei die Verarbeitungsschaltung für Folgendes konfiguriert ist:
Authentifizieren einer Identifizierung eines Benutzers der mobilen Rechenvorrichtung (402, 422); und
Übermitteln einer elektronischen Autorisierung von der mobilen Rechenvorrichtung (402, 422) an eine Bereitstellungsvorrichtung (404, 424) unter Verwendung eines ersten Kommunikationskanals;
wobei die elektronische Autorisierung der Bereitstellungsvorrichtung (404, 424) Zugriff auf elektronische Gesundheitsakten bereitstellt, die in einem Container auf dem im Besitz des Benutzers befindlichen Netzwerkserver gespeichert sind;
wobei der Zugriff auf die elektronischen Gesundheitsakten des Benutzers über einen zweiten Kommunikationskanal bereitgestellt wird, der sich vom ersten Kommunikationskanal unterscheidet;
wobei der zweite Kommunikationskanal ein Weitverkehrsnetzwerk umfasst, das konfiguriert ist, um Zugriff auf den Container auf dem Netzwerkserver bereitzustellen;
wobei die elektronischen Gesundheitsakten des Benutzers verschlüsselt und in dem Container abgelegt sind; und
wobei die in dem Container abgelegten elektronischen Gesundheitsakten vom Netzwerkserver nach einer ersten Datenrückgewinnung der elektronischen Gesundheitsakten des Benutzers aus dem Container gelöscht werden.

10. System nach Anspruch 9, wobei die Verarbeitungsschaltung konfiguriert ist, um eine elektronische Autorisierung von der mobilen Rechenvorrichtung (402,
422) an eine Bereitstellungsvorrichtung durch Folgendes zu übermitteln:
Bewirken, dass ein codiertes Bild auf einer Anzeige der mobilen Rechenvorrichtung (402, 422) angezeigt wird, wobei das codierte Bild Informationen bereitstellt, die den Benutzer der mobilen Vorrichtung (402, 422) und eine Adresse der elektronischen Gesundheitsakten des Benutzers identifizieren, wenn das codierte Bild von einer Kamera der Bereitstellungsvorrichtung (404, 424) erfasst und durch die Bereitstellungsvorrichtung (404, 424) decodiert wird.

11. System nach Anspruch 10, wobei das codierte Bild einen Schnellantwortcode oder einen Barcode umfasst und kryptografische Schlüssel einschließt, und das Bild von der mobilen Rechenvorrichtung (402, 422) zur Erfassung durch eine Kamera der Bereitstellungsvorrichtung (404, 424) angezeigt wird, und wobei die Bereitstellungsvorrichtung (404, 424) konfiguriert ist, um die kryptografischen Schlüssel zu verwenden, um auf die elektronischen Gesundheitsakten des Benutzers zuzugreifen.

12. System nach Anspruch 9, wobei die elektronische Autorisierung unter Verwendung eines Nahfeldkommunikationsprotokolls, eines Bluetooth-Protokolls oder eines Zigbee-Protokolls übermittelt wird.

13. System nach Anspruch 9, wobei die in dem Container abgelegten elektronischen Gesundheitsakten nach einer vorbestimmten Zeit gelöscht werden, falls sie nicht aus dem Container rückgewonnen werden.

14. System nach Anspruch 13, wobei mindestens eine/r der mobilen Rechenvorrichtung (402, 422), der Bereitstellungsvorrichtung (404, 424) und des Netzwerkservers ein Protokoll führt, das sich auf Transaktionen bezieht, die den Container einschließen, und wobei das Protokoll eine oder mehrere einer Beschreibung der in dem Container abgelegten elektronischen Gesundheitsakten, der Identität des Benutzers des mobilen Rechenvorrichtung (402, 422) oder einer Identität der Bereitstellungsvorrichtung aufzeichnet, wenn die Bereitstellungsvorrichtung (404, 424) auf den Container zugreift.

## Revendications

1. Procédé permettant de commander l'accès à des dossiers médicaux électroniques, stockés dans un conteneur sur un serveur réseau, le procédé
comprenant :
l'authentification (702) d'une identification d'un utilisateur d'un dispositif mobile (402, 422), l'utilisateur étant un propriétaire des dossiers médicaux électroniques, et ledit dispositif mobile (402, 422) étant un premier dispositif informatique mobile (402, 422) ; et
la communication d'une autorisation électronique depuis le dispositif mobile (402, 422) à un dispositif fournisseur (404, 424) à l'aide d'un premier canal de communication ;
ladite autorisation électronique permettant au dispositif fournisseur (404, 424) d'accéder aux dossiers de soins médicaux électroniques de l'utilisateur ;
ledit accès aux dossiers de soins médicaux électroniques de l'utilisateur étant fourni par un second canal de communication qui est différent du premier canal de communication ; et
ledit second canal de communication comprenant un réseau étendu qui est configuré pour fournir un accès au conteneur sur le serveur de réseau,
lesdits dossiers de soins médicaux électroniques de l'utilisateur étant chiffrés et déposés dans le conteneur, et les dossiers de soins médicaux électroniques déposés dans le conteneur étant supprimés après une première récupération des dossiers de soins médicaux électroniques de l'utilisateur du conteneur.

2. Procédé selon la revendication 1, ladite communication d'une autorisation électronique depuis le dispositif mobile (402, 422) à un dispositif fournisseur comprenant :
le transfert d'une image codée du dispositif mobile (402, 422) au dispositif fournisseur (404, 424) après que l'identification de l'utilisateur du dispositif mobile (402, 422) a été authentifiée.

3. Procédé selon la revendication 2, ledit transfert de l'image codée du dispositif mobile (402, 422) au dispositif fournisseur (404, 424) comprenant :
l'affichage (708) de l'image codée sur un dispositif d'affichage du dispositif mobile (402, 422), ladite image codée fournissant des informations identifiant l'utilisateur du dispositif mobile (402, 422) et une adresse des dossiers de soins médicaux électroniques de l'utilisateur lorsque l'image codée est capturée (752) par une caméra du dispositif fournisseur et décodée par le dispositif fournisseur (404, 424).

4. Procédé selon la revendication 3, ledit dispositif fournisseur (404, 424) étant configuré pour utiliser des clés cryptographiques fournies dans l'image codée pour accéder (756) aux dossiers de soins médicaux électroniques de l'utilisateur.

5. Procédé selon la revendication 2, ledit premier canal de communication comprenant une liaison vidéo par l'intermédiaire d'un réseau connectant le dispositif mobile (402, 422) et le dispositif fournisseur.

6. Procédé selon la revendication 1, ledit premier canal de communication étant commandé par un protocole de communication en champ proche, un protocole Bluetooth ou un protocole Zigbee.

7. Procédé selon la revendication 1, lesdits dossiers de soins médicaux électroniques déposés dans le conteneur étant supprimés après un temps prédéfini s'ils ne sont pas récupérés du conteneur.

8. Procédé selon la revendication 7, au moins l'un du dispositif mobile (402, 422), du dispositif fournisseur (404, 424) et du serveur réseau maintenant un journal lié aux transactions impliquant le conteneur, et ledit journal enregistrant un ou plusieurs d'une description des dossiers de soins médicaux électroniques déposés dans le conteneur, de l'identité de l'utilisateur du dispositif mobile (402, 422) ou d'une identité du dispositif fournisseur (404, 424) lorsque le dispositif fournisseur (404, 424) accède au conteneur.

9. Système comprenant un dispositif informatique mobile (402, 422) et un serveur réseau, ledit dispositif informatique mobile (402, 422) étant configuré pour des communications sans fil et comprenant :
un circuit de traitement, ledit circuit de traitement étant configuré pour :
authentifier une identification d'un utilisateur du dispositif informatique mobile (402, 422) ; et
communiquer une autorisation électronique depuis le dispositif informatique mobile (402, 422) à un dispositif fournisseur (404, 424) à l'aide d'un premier canal de communication ;
ladite autorisation électronique permettant au dispositif fournisseur (404, 424) d'accéder aux dossiers de soins médicaux électroniques stockés dans un conteneur sur le serveur réseau détenu par l'utilisateur ;
ledit accès aux dossiers de soins médicaux électroniques de l'utilisateur étant fourni par un second canal de communication qui est différent du premier canal de communication ;
ledit second canal de communication comprenant un réseau étendu qui est configuré pour fournir un accès au conteneur sur le serveur réseau ;
lesdits dossiers de soins médicaux électroniques de l'utilisateur étant chiffrés et déposés dans le conteneur ; et
lesdits dossiers de soins médicaux électroniques déposés dans le conteneur étant supprimés par le serveur réseau, après une première récupération des dossiers de soins médicaux électroniques de l'utilisateur, du conteneur.

10. Système selon la revendication 9, ledit circuit de traitement étant configuré pour communiquer une autorisation électronique à partir du dispositif informatique mobile (402, 422) à un dispositif fournisseur en :
amenant une image codée à être affichée sur un dispositif d'affichage du dispositif informatique mobile (402, 422), ladite image codée fournissant des informations identifiant l'utilisateur du dispositif mobile (402, 422) et une adresse des dossiers de soins médicaux électroniques de l'utilisateur lorsque l'image codée est capturée par une caméra du dispositif fournisseur (404, 424) et décodée par le dispositif fournisseur (404, 424).

11. Système selon la revendication 10, ladite image codée comprenant un code de réponse rapide ou un code à barres et comprenant des clés cryptographiques et ladite image étant affichée par le dispositif informatique mobile (402, 422) en vue d'une capture par une caméra du dispositif fournisseur (404, 424), et ledit dispositif fournisseur (404, 424) étant configuré pour utiliser les clés cryptographiques pour accéder aux dossiers de soins médicaux électroniques de l'utilisateur.

12. Système selon la revendication 9, ladite autorisation électronique étant communiquée à l'aide d'un protocole de communication en champ proche, d'un protocole Bluetooth ou d'un protocole Zigbee.

13. Système selon la revendication 9, lesdits dossiers de soins médicaux électroniques déposés dans le conteneur étant supprimés, après un temps prédéfini s'ils ne sont pas récupérés, du conteneur.

14. Système selon la revendication 13, au moins l'un du dispositif informatique mobile (402, 422), du dispositif fournisseur (404, 424) et du serveur réseau maintenant un journal lié aux transactions impliquant le conteneur, et ledit journal enregistrant l'un ou plusieurs d'une description des dossiers de soins médicaux électroniques déposés dans le conteneur, de l'identité de l'utilisateur du dispositif informatique mobile (402, 422), ou d'une identité du dispositif fournisseur lorsque le dispositif fournisseur (404, 424) accède au conteneur.
